(19) Europäisches Patentamt
European Patent Office
Office européen des brevets

(11) EP 1 892 293 A1

(12) EUROPEAN PATENT APPLICATION
published in accordance with Art. 153(4) EPC

(43) Date of publication:
27.02.2008 Bulletin 2008/09

(21) Application number: 06757008.5

(22) Date of filing: 06.06.2006

(51) Int Cl.:
*C12N 15/09* (2006.01) *A61K 31/713* (2006.01)
*A61P 1/04* (2006.01) *A61P 9/00* (2006.01)
*A61P 9/10* (2006.01) *A61P 11/00* (2006.01)
*A61P 11/06* (2006.01) *A61P 13/02* (2006.01)
*A61P 13/12* (2006.01) *A61P 17/00* (2006.01)
*A61P 17/02* (2006.01) *A61P 17/06* (2006.01)
*A61P 19/02* (2006.01) *A61P 29/00* (2006.01)
*A61P 35/00* (2006.01) *A61P 35/04* (2006.01)
*A61P 37/00* (2006.01) *A61P 37/08* (2006.01)
*A61P 41/00* (2006.01) *A61P 43/00* (2006.01)
*C07H 21/00* (2006.01)

(86) International application number:
PCT/JP2006/311268

(87) International publication number:
WO 2006/132204 (14.12.2006 Gazette 2006/50)

(84) Designated Contracting States:
AT BE BG CH CY CZ DE DK EE ES FI FR GB GR HU IE IS IT LI LT LU LV MC NL PL PT RO SE SI SK TR

(30) Priority: 06.06.2005 JP 2005165477

(71) Applicant: AnGes MG, Inc.
Ibaraki-shi,
Osaka 567-0085 (JP)

(72) Inventors:
- GEMBA, Takefumi
6660014 (JP)
- UENO, Nobuhiro
5691025 (JP)

(74) Representative: Gille Hrabal Struck Neidlein Prop Roos
Patentanwälte
Bruecknerstrasse 20
40593 Düsseldorf (DE)

# (54) TRANSCRIPTION FACTOR DECOY

(57) Double-stranded oligonucleotides useful as decoy oligonucleotides having a high binding ability to a transcription factor and having a reduced cytotoxicity are disclosed. Each of the double-stranded oligonucleotides is formed by hybridization of a sense strand oligonucleotide of the following Formula A:

5'-N(m)-G-Consensus Sequence -C-N(n)-3' (Formula A)

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represent(s) nucleotide A, G, T, C or U; m and n represent each independently an integer of 0 to 20; and Consensus Sequence represents a consensus sequence to which a transcription factor binds)
and an antisense strand oligonucleotide complementary to said sense strand oligonucleotide.

EP 1 892 293 A1

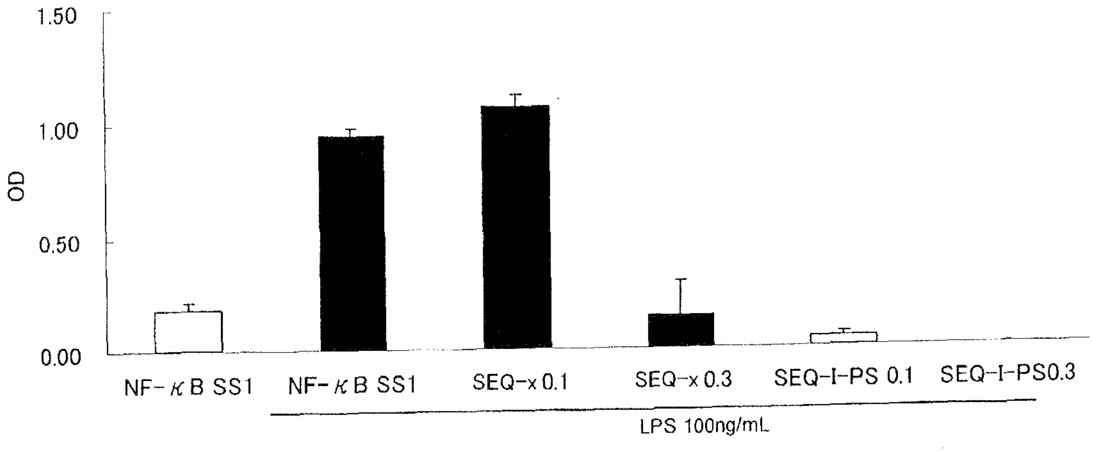
1.50
1.00
OD
0.50
0.00
NF-κB SS1
NF-κB SS1
SEQ-x 0.1
SEQ-x 0.3
SEQ-I-PS 0.1
SEQ-I-PS0.3
LPS 100ng/mL

Fig.1

SEQUENCE LISTING

<110> ANGES MG, Inc.

<120> transcription factor decoys

<130> 06PF0319-PCT

<160> 43

<170> PatentIn version 3.1

<210> 1
<211> 10
<212> DNA
<213> Artificial

<220>
<223> consensus sequence

<400> 1
gggrhtyyhc 10

<210> 2
<211> 10
<212> DNA
<213> Artificial

<220>
<223> consensus sequence

<400> 2
gggatttccc 10

<210> 3
<211> 10
<212> DNA
<213> Artificial

<220>
<223> consensus sequence

<400> 3
gggactttcc 10

<210> 4
<211> 20
<212> DNA
<213> Artificial

<220>
<223> oligonucleotide

<400> 4
ccttgagggg atttcccccc 20

<210> 5
<211> 10
<212> DNA

<213> Artificial

<220>
<223> consensus sequence

<220>
<221> misc_feature
<222> (4)..(7)
<223> "n" represents A, G, T or C

<400> 5
ttcnnnngaa 10

<210> 6
<211> 10
<212> DNA
<213> Artificial

<220>
<223> consensus sequence

<400> 6
ttcccaagaa 10

<210> 7
<211> 20
<212> DNA
<213> Artificial

<220>
<223> oligonucleotide

<400> 7
ccttgaaggg atttccctcc 20

<210> 8
<211> 14
<212> DNA
<213> Artificial

<220>
<223> oligonucleotide

<400> 8
gaggggattt cccc 14

<210> 9
<211> 16
<212> DNA
<213> Artificial

<220>
<223> oligonucleotide

<400> 9
ttgaggggat ttcccc 16

<210> 10
<211> 17
<212> DNA
<213> Artificial

<220>
<223> oligonucleotide

<400> 10
tttgagggga tttcccc 17

<210> 11
<211> 20
<212> DNA
<213> Artificial

<220>
<223> oligonucleotide

<400> 11
ccttgaaggg actttcctcc 20

<210> 12
<211> 22
<212> DNA
<213> Artificial

<220>
<223> oligonucleotide

<400> 12
agttgagggg atttccccag gc 22

<210> 13
<211> 23
<212> DNA
<213> Artificial

<220>
<223> oligonucleotide

<400> 13
agtatcgggg atttcccctt aac 23

<210> 14
<211> 20
<212> DNA
<213> Artificial

<220>
<223> oligonucleotide

<400> 14
agctggggat tcccctttg 20

<210> 15
<211> 23
<212> DNA

```
<213>  Artificial

<220>
<223>  oligonucleotide

<400>  15
accaggggat ttccccgtcc cac                                               23


<210>  16
<211>  20
<212>  DNA
<213>  Artificial

<220>
<223>  oligonucleotide

<400>  16
agttgagggg atttccccgc                                                   20


<210>  17
<211>  20
<212>  DNA
<213>  Artificial

<220>
<223>  oligonucleotide

<400>  17
acttgaaggg atttccctcc                                                   20


<210>  18
<211>  20
<212>  DNA
<213>  Artificial

<220>
<223>  oligonucleotide

<400>  18
ccutgagggg atttcccccc                                                   20


<210>  19
<211>  20
<212>  DNA
<213>  Artificial

<220>
<223>  oligonucleotide

<400>  19
ccuugagggg atttcccccc                                                   20


<210>  20
<211>  20
<212>  DNA
<213>  Artificial

<220>
```

<223> oligonucleotide

<400> 20
ccttgagggg attucccccc 20

<210> 21
<211> 12
<212> DNA
<213> Artificial

<220>
<223> oligonucleotide

<400> 21
ggggatttcc cc 12

<210> 22
<211> 13
<212> DNA
<213> Artificial

<220>
<223> oligonucleotide

<400> 22
aggggatttc ccc 13

<210> 23
<211> 15
<212> DNA
<213> Artificial

<220>
<223> oligonucleotide

<400> 23
tgaggggatt tcccc 15

<210> 24
<211> 17
<212> DNA
<213> Artificial

<220>
<223> oligonucleotide

<400> 24
cttgagggga tttcccc 17

<210> 25
<211> 18
<212> DNA
<213> Artificial

<220>
<223> oligonucleotide

<400> 25

ccttgagggg atttcccc 18

<210> 26
<211> 13
<212> DNA
<213> Artificial

<220>
<223> oligonucleotide

<400> 26
ggggatttcc ccc 13

<210> 27
<211> 14
<212> DNA
<213> Artificial

<220>
<223> oligonucleotide

<400> 27
aggggatttc cccc 14

<210> 28
<211> 15
<212> DNA
<213> Artificial

<220>
<223> oligonucleotide

<400> 28
gaggggattt ccccc 15

<210> 29
<211> 16
<212> DNA
<213> Artificial

<220>
<223> oligonucleotide

<400> 29
tgaggggatt tccccc 16

<210> 30
<211> 17
<212> DNA
<213> Artificial

<220>
<223> oligonucleotide

<400> 30
ttgaggggat ttccccc 17

<210> 31
<211> 18
<212> DNA
<213> Artificial

<220>
<223> oligonucleotide

<400> 31
cttgaggggga tttccccc 18

<210> 32
<211> 19
<212> DNA
<213> Artificial

<220>
<223> oligonucleotide

<400> 32
ccttgagggg atttccccc 19

<210> 33
<211> 14
<212> DNA
<213> Artificial

<220>
<223> oligonucleotide

<400> 33
ggggatttcc cccc 14

<210> 34
<211> 15
<212> DNA
<213> Artificial

<220>
<223> oligonucleotide

<400> 34
aggggatttc ccccc 15

<210> 35
<211> 16
<212> DNA
<213> Artificial

<220>
<223> oligonucleotide

<400> 35
gaggggattt cccccc 16

<210> 36
<211> 17
<212> DNA

<213> Artificial

<220>
<223> oligonucleotide

<400> 36
tgaggggatt tcccccc 17

<210> 37
<211> 18
<212> DNA
<213> Artificial

<220>
<223> oligonucleotide

<400> 37
ttgaggggat ttcccccc 18

<210> 38
<211> 19
<212> DNA
<213> Artificial

<220>
<223> oligonucleotide

<400> 38
cttgagggga tttcccccc 19

<210> 39
<211> 22
<212> DNA
<213> Artificial

<220>
<223> oligonucleotide

<400> 39
agttgaaggg atttccctag gc 22

<210> 40
<211> 20
<212> DNA
<213> Artificial

<220>
<223> oligonucleotide

<400> 40
ccttgagggg actttccccc 20

<210> 41
<211> 23
<212> DNA
<213> Artificial

<220>

```
<223>  oligonucleotide

<400>  41
accaagggat ttccctgtcc cac                                              23


<210>  42
<211>  22
<212>  DNA
<213>  Artificial

<220>
<223>  NF-kB decoy

<400>  42
agttgagggg actttcccag gc                                               22


<210>  43
<211>  21
<212>  DNA
<213>  Artificial

<220>
<223>  NF-kB decoy

<400>  43
gatcgagggg actttcccta g                                                21
```

## Description

Technical Field

The present invention relates to an oligonucleotide effective for modulating gene expression in living bodies, as well as to the uses thereof and pharmaceutical compositions containing the same.

Background Art

Recently, as therapeutic and prophylactic methods for a specific disease, methods in which expression of a specific gene is controlled using a nucleic acid or a derivative thereof are now being developed. For example, in cases where a disease can be treated or prevented by suppressing the expression of a gene related to the onset of the disease, therapy or prophylaxis of the disease is attained by administering a nucleic acid or a derivative thereof which can specifically suppress the expression of the gene to the living body or the site of the subject to be treated.

Representative examples of such methods include the antisense method and decoy oligonucleotide method. The former is the method in which a single-stranded antisense oligonucleotide against the translation product of the specific gene (i.e., mRNA) is delivered into cells to suppress the transcription of the specific gene.
The latter decoy oligonucleotide method is a method in which a double-stranded oligonucleotide to which a transcription factor that induces the expression of the gene related to the onset of the disease is delivered into cells to inhibit the binding of the transcription factor to the binding site on the genome, thereby suppressing the expression of the gene induced by the transcription factor. The term decoy is an English word which means "decoy", and one having the structure similar to that which a substance binds to or acts on is called a decoy.

The mechanism of the decoy oligonucleotide method is, briefly, as follows: That is, for the purpose of suppressing expression of the gene induced by a specific transcription factor, a double-stranded oligonucleotide targeting the specific transcription factor is delivered to the subject tissue or cells. The double-stranded oligonucleotide then moves into the each nucleus and competes in the binding of the transcription factor to the binding site of the transcription factor on the genome in the nucleus, so that the binding between the binding site of the transcription factor on the genome and the transcription factor is inhibited. Therefore, expression of the gene induced by the transcription factor is suppressed. As a result, the biological phenomenon by the expression of the gene induced by the transcription factor is inhibited.

Thus, it is important for the decoy oligonucleotide used as a prophylactic or therapeutic agent for a disease that the oligonucleotide has a high binding activity to the target transcription factor in order to make the above-described mechanism effectively work, in addition to that the oligonucleotide has a high binding specificity to the target transcription factor and that the cytotoxicity of the oligonucleotide is low.

In the designing of the decoy oligonucleotide, it is generally thought advantageous that the decoy oligonucleotide contain a consensus sequence to which the transcription factor binds. It is known that decoy oligonucleotides thus designed against various transcription factors are effective for many medical uses (see, for example, Japanese PCT Patent Application Re-laid-open No. 96/035430, JP 3392143 B, WO95/11687 and so on).

It is well-known that it is also an important key for making the above-described mechanism effectively work that the decoy oligonucleotide delivered into the cells can exist stably in the cells for a long time. Since oligonucleotides are decomposed by nucleases in the cells, it is difficult to make the oligonucleotides stably exist in the cells and in the nuclei. To overcome this difficult problem, methods in which various modifications are given to the bases in the oligonucleotides have been tried (for example, Milligan et al., J. Med. Chem. 1993, 36, 1923, and Japanese Translated PCT Patent Application Laid-open No. 08-501928). Among these modifications, the most frequently used modification is the modification by phosphorothioate (PS). Since phosphorothioated oligonucleotides are resistant to nucleases, they draw attention as oligonucleotides for therapies.

However, while phosphorothioated oligonucleotides have much higher resistance to nucleases than the natural phosphodiester oligonucleotides, the disadvantages that the binding ability to the target molecule is decreased when compared with the phosphodiester oligonucleotides, and the specificity to the target molecule is decreased are observed in many cases (Milligan et al., J. Med. Chem. 1993, 36, 1923; Stein & Cheng, Science 1993, 261, 1004). Further, since phosphorothioate group is toxic, in many cases, phosphorothioated oligonucleotides have higher cytotoxicity than phosphodiester oligonucleotides (Levin et al., Biochem. Biophys. Acta, 1999, 1489, 69). This is also a disadvantage of the phosphorothioated oligonucleotides when used as therapeutic agents.

The present inventors have discovered that NF-κB decoy oligonucleotides composed of an oligonucleotide having a sequence containing a binding sequence of NF-κB and its complementary strand are useful for therapies of diseases related to NF-κB (WO96/35430, WO02/066070, WO03/043663, WO03/082331, WO03/099339, WO04/026342, WO05/004913 and WO05/004914).

Patent Literature 1: Japanese PCT Patent Application Re-laid-open No. 96/035430
Patent Literature 2: JP 3392143 B
Patent Literature 3: WO95/11687
Patent Literature 4: Japanese Translated PCT Patent Application Laid-open No. 08-501928
Patent Literature 5: WO96/35430
Patent Literature 6: WO02/066070
Patent Literature 7: WO03/043663
Patent Literature 8: WO03/082331
Patent Literature 9: WO03/099339
Patent Literature 10: WO04/026342
Patent Literature 11: WO05/00491 3
Patent Literature 12: WO05/004914
Non-patent Literature 1: Milligan et al., J. Med. Chem. 1993, 36, 1923
Non-patent Literature 2: Levin et al., Biochem. Biophys. Acta, 1999, 1489, 69
Non-patent Literature 3: FASEB J 2002 Nov 16(13) 1811-3 Epub 2002 Sep 5
Non-patent Literature 4: Naunyu-Schmiedeberg's Arch Pharmacol (2001) 364 422-429

Disclosure of the Invention

Problems to be Solved by the Invention

An object of the present invention is to provide a double-stranded oligonucleotide useful as a decoy oligonucleotide, which is more effective, that is, which has a higher binding activity to a transcription factor and which has a reduced cytotoxicity, as well as a medical use thereof.

Means for Solving the Problem

To solve the above-described problems, the present inventors intensively studied to develop a decoy oligonucleotide having an increased binding activity to a transcription factor by selecting the bases in the flanking sequence, which bases are adjacent to the consensus sequence in accordance with a specific rule, and further to develop a modified decoy oligonucleotide whose stability in the cells is increased and the cytotoxicity brought about by the chemical modification of a natural oligonucleotide is decreased, thereby completing the present invention.
Accordingly, the present invention relates to the following:

1. A double-stranded oligonucleotide formed by hybridization of a sense strand oligonucleotide of the following Formula A:

5'-N(m)-G-Consensus Sequence -C-N(n)-3' (Formula A)

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; and the Consensus Sequence represents a consensus sequence to which a transcription factor binds) and an antisense strand oligonucleotide complementary to the sense strand oligonucleotide (excluding those oligonucleotides wherein the sense strand oligonucleotide thereof is the sequence AGTTGAGGGGACTTTCCCAGGC (SEQ ID NO:42) or GATCGAGGGGACTTTCCCTAG (SEQ ID NO:43)).

2. The oligonucleotide according to 1 described above, wherein the m and n are integers of not more than 6.
3. The oligonucleotide according to 1 or 2 described above, wherein in the Formula A, the m is an integer of 2 to 6.
4. The oligonucleotide according to 2 or 3 described above, wherein in the Formula A, n is an integer of 0, 1, 2 or 3.

5. A double-stranded oligonucleotide formed by hybridization of a sense strand oligonucleotide of the following

Formula A:

$$5'\text{-N(m)-G-Consensus Sequence -C-N(n)-3'} \qquad \text{(Formula A)}$$

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; the Consensus Sequence means the consensus sequence to which a transcription factor binds; and the N (m) is a sequence selected from the group consisting of GA, TGA, TTGA, CTTGA and CCTTGA) and an antisense strand oligonucleotide complementary to the sense strand oligonucleotide.

6. The oligonucleotide according to 4 or 5 described above, wherein in the Formula A, N(n) is C or CC.
7. The oligonucleotide according to 6 described above, wherein in the Formula A, N(m) is an oligonucleotide whose sequence is CCTTGA, and N(n) is an oligonucleotide whose sequence is CC.
8. The oligonucleotide according to any one of 1 to 7 described above, wherein bonds between bases comprise a phosphorothioate bond.
9. The oligonucleotide according to 8 described above, wherein 3 bases at an end(s) of at least one of the sense strand and antisense strand are bound through phosphorothioate bonds.
10. The oligonucleotide according to 8 described above, wherein 3 bases each at both ends of the sense strand and the antisense strand comprise a phosphorothioate bond, but other than these, none of the strands comprises adjacent base pairs bound through a phosphorothioate bond.
11. The oligonucleotide according to any one of 8 to 10 described above, wherein 4 or more consecutive phosphorothioate bonds do not exist in any of the sense strand and the antisense strand.
12. The oligonucleotide according to any one of 8 to 11 described above, wherein 2 or 3 consecutive phosphorothioate bonds exist in each of the sense strand and the antisense strand.
13. The oligonucleotide according to any one of 8 to 12 described above, wherein the bond between the two bases at the 5'-end is a phosphorothioate bond in both of the sense strand and the antisense strand, respectively.
14. The oligonucleotide according to 13 described above, wherein the bonds between 3 bases at the 5'-end are phosphorothioate bonds in both of the sense strand and the antisense strand, respectively.
15. The oligonucleotide according to any one of 8 to 14 described above, wherein the bond between 2 bases at the 3'-end is a phosphorothioate bond in both of the sense strand and the antisense strand, respectively.
16. The oligonucleotide according to 15 described above, further characterized in that the bonds between 3 bases at the 3'-end are phosphorothioate bonds in both of the sense strand and the antisense strand, respectively.
17. The oligonucleotide according to any one of 1 to 16 described above, wherein the consensus sequence is a binding sequence of NF-κB, E2F, GATA-3, STAT-1, STAT-6, Ets or AP-1.
18. The oligonucleotide according to 17 described above, wherein the consensus sequence is a binding sequence of NF-κB, the consensus sequence being represented by GGGRHTYYHC (wherein R represents A or G; Y represents C or T; H represents A, C or T) (SEQ ID NO:1).
19. The oligonucleotide according to 18 described above, wherein the binding sequence of NF-κB is GGGATTTCCC (SEQ ID NO:2) or GGGACTTTCC (SEQ ID NO:3).
20. The oligonucleotide according to 20 described above, wherein the sense strand oligonucleotide has a sequence CCTTGAGGGGATTTCCCCCC (SEQ ID NO:4).
21. The oligonucleotide according to 20 described above, wherein among the bonds between bases in the sense strand CCTTGAGGGGATTTCCCCCC (SEQ ID NO:4), only the first, second, 5th, 6th, 14th and 15th bonds from the 5'-end are phosphorothioate bonds, and among the bonds between bases in the antisense strand, only the first, second, 9th, 10th and 11th bonds from the 5'-end thereof are phosphorothioate bonds.

22. A double-stranded oligonucleotide formed by hybridization of a sense strand oligonucleotide of the following Formula C:

$$5'\text{-}N(x)\text{-Consensus Sequence-}N(y)\text{-}3' \qquad \text{Formula C}$$

(wherein N(x) is a flanking sequence at the 5'-end, and represents that "N"(s) in the number of x is(are) ligated; N (y) is a flanking sequence at the 3'-end, and represents that "N"(s) in the number of y is(are) ligated; all "N"s each independently represents nucleotide A, G, T, C or U; m and n represent each independently an integer of 1 to 20; and Consensus Sequence represents a consensus sequence to which a transcription factor binds)
and an antisense strand oligonucleotide complementary to the sense strand oligonucleotide, the antisense strand oligonucleotide comprising consecutive 4 bases wherein the bonds therebetween are phosphorothioate bonds, the consecutive 4 bases not including the base at an end of the oligonucleotide.
23. The nucleotide according to 22 described above, wherein only one set of the consecutive 4 bases is contained in the antisense strand oligonucleotide.
24. The nucleotide according to 23 described above, wherein only one set of the consecutive 4 bases is contained in the double-stranded nucleotide.
25. The nucleotide according to any one of 1 to 24 described above, wherein Consensus Sequence in the Formula C is a binding sequence of NF-κB, E2F, GATA-3, STAT-1, STAT-6, Ets or AP-1.
26. The oligonucleotide according to 25 described above, wherein the consensus sequence is a binding sequence of NF-κB, the consensus sequence being represented by GGGRHTYYHC (wherein R represents A or G; Y represents C or T; H represents A, C or T) (SEQ ID NO: 1).
27. The oligonucleotide according to 26 described above, wherein the binding sequence of NF-κB is GGGATTTCCC (SEQ ID NO:2) or GGGACTTTCC (SEQ ID NO:3).

28. The oligonucleotide according to any one of 18 to 27 described above, wherein the Formula C is represented by the following Formula A:

$$5'\text{-}N(m)\text{-G-Consensus Sequence -C-}N(n)\text{-}3' \qquad \text{(Formula A)}$$

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; and the Consensus Sequence represents a consensus sequence to which a transcription factor binds).
29. The oligonucleotide according to 28 described above, wherein in the Formula A, the m and n are integers of not more than 6.
30. The oligonucleotide according to 29 described above, wherein in the Formula A, the m is an integer of 2 to 6.
31. The oligonucleotide according to any one of 22 to 30 described above, wherein in the Formula A, n is an integer of 0, 1, 2 or 3.
32. The oligonucleotide according to 30 or 31 described above, wherein in the Formula A, N(m) is selected from the group consisting of GA, TGA, TTGA, CTTGA and CCTTGA.
33. The oligonucleotide according to 32 described above, wherein in the Formula A, N(m) is GA or TTGA, and n is 0.
34. The oligonucleotide according to 33 described above, wherein the sense strand oligonucleotide is GAGGGGATTTCCCC (SEQ ID NO:8).
35. The oligonucleotide according to 34 described above, wherein among bonds between bases in the sense strand GAGGGGATTTCCCC (SEQ ID NO:8), only the first, second, 10th and 11th bonds from the 5'-end are phosphorothioate bonds, and among bonds between bases in the antisense strand, only the first, 7th, 8th and 9th bonds from the 5'-end are phosphorothioate bonds.

36. A transcription factor inhibitor comprising the oligonucleotide according to any one of 1 to 35 described above.
37. The transcription factor inhibitor according to 36 described above, wherein the transcription factor is NF-κB.
38. Use of the oligonucleotide according to any one of 1 to 35 described above for the production of a transcription factor inhibitor.
39. The use according to 38 described above, wherein the transcription factor is NF-κB.

40. A method for inhibiting transcription factor in a living body, the method comprising administering to the living body an effective amount of the oligonucleotide according to any one of 1 to 35 described above.

41. The method according to 40 described above, wherein the transcription factor is NF-κB.

42. A pharmaceutical composition comprising as an effective ingredient the oligonucleotide according to any one of 1 to 35 described above.

43. An agent for prophylaxis, amelioration and/or therapy of ischemic diseases, allergic diseases, inflammatory diseases, autoimmune diseases, metastasis/infiltration of cancers, or cachexy, the agent comprising as an effective ingredient the oligonucleotide according to any one of 1 to 35 described above.

44. An agent for prophylaxis, amelioration and/or therapy of vascular restenosis, acute coronary syndrome, brain ischemia, myocardial infarction, reperfusion hindrance of ischemic diseases, atopic dermatitis, psoriasis vulgaris, contact dermatitis, keloid, decubital ulcer, ulcerative colitis, Crohn's disease, nephropathy, glomerulosclerosis, albuminuria, nephritis, renal failure, rheumatoid arthritis, osteoarthritis, degenerative intervertebral disc, asthma, chronic obstructive pulmonary disease (COPD) or cystic fibrosis (CF), the agent comprising as an effective ingredient the oligonucleotide according to any one of 1 to 35 described above.

45. An agent for prophylaxis, amelioration and/or therapy of vascular restenosis which occurs after PTCA (percutaneous transluminal coronary angioplasty), PTA (percutaneous transluminal angioplasty), bypass surgery, organ transplantation or surgery of an organ, the agent comprising as an effective ingredient the oligonucleotide according to any one of 1 to 35 described above.

46. The agent for prophylaxis, amelioration and/or therapy according to 45 described above, wherein the vascular restenosis is caused by using an artificial blood vessel, catheter or stent, or by vein grafting.

47. The agent for prophylaxis, amelioration and/or therapy according to 45 described above, wherein the vascular restenosis is caused by a surgical treatment for arteriosclerosis obliterans, aneurysm, aorta dissection, acute coronary syndrome, brain ischemia, Marfan syndrome or plaque rupture.

48. A pharmaceutical composition for prophylaxis, amelioration and/or therapy of a disease(s) caused by NF-κB, the pharmaceutical composition comprising as an effective ingredient the oligonucleotide according to any one of 18 to 21, and 25 to 27 described above.

49. The pharmaceutical composition according to 48 described above, wherein the disease(s) caused by NF-κB is (are) at least one selected from the group consisting of ischemic diseases, allergic diseases, inflammatory diseases, autoimmune diseases, metastasis/infiltration of cancers and cachexy.

50. Use of the oligonucleotide according to any one of 1 to 35 described above for the production of a pharmaceutical composition.

51. Use of the oligonucleotide according to any one of 18 to 21, and 25 to 27 for the production of an agent for therapy or prophylaxis of a disease(s) caused by NF-κB.

52. The use according to 51 described above, wherein the disease(s) caused by NF-κB is(are) at least one selected from the group consisting of ischemic diseases, allergic diseases, inflammatory diseases, autoimmune diseases, metastasis/infiltration of cancers and cachexy.

53. A method for therapy and/or prophylaxis of a disease(s) caused by NF-κB, the method comprising administering to a patient or an animal in need of such therapy and/or prophylaxis an effective amount of the oligonucleotide according to any one of 18 to 21, and 25 to 27 described above.

54. The method according to 53 described above, wherein the disease(s) caused by NF-κB is(are) at least one selected from the group consisting of ischemic diseases, allergic diseases, inflammatory diseases, autoimmune diseases, metastasis/infiltration of cancers and cachexy.

Effects of the Invention

By the present invention, a double-stranded oligonucleotide useful as a decoy oligonucleotide, which has a higher binding activity to a transcription factor and which has a reduced cytotoxicity, as well as a medical use thereof, was first provided. Since the double-stranded oligonucleotide according to the present invention has a high stability in a living body, can effectively inhibit the transcription factor and has a low toxicity, it is useful for inhibiting the transcription activity, and in turn, exhibits excellent performance in the medical uses brought about by the inhibition of the transcription factor.

Brief Description of the Drawings

Fig. 1 is a graph showing the results of an intracellular NF-κB binding test.
Fig. 2 shows the results of a toxicity test of cells.
Fig. 3 shows the image of polyacrylamide gel electrophoresis, obtained in a stability test. Staining of the gel was performed with ethidium bromide.

Best Mode for Carrying out the Invention

According to the first aspect of the present invention, the present invention provides a double-stranded oligonucleotide formed by hybridization of a sense strand oligonucleotide of the following Formula A:

5'-N(m)-G-Consensus Sequence -C-N(n)-3' (Formula A)

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20) and an antisense strand oligonucleotide complementary to the sense strand oligonucleotide (excluding those oligonucleotides wherein the sense strand oligonucleotide thereof is the sequence AGTTGAGGGGACTTTCCCAGGC (SEQ ID NO:42) or GATCGAGGGGACTTTCCCTAG (SEQ ID NO:43)). The oligonucleotides whose sequences are shown in SEQ ID NO:42 and SEQ ID NO:43, respectively, are the known NF-κB decoys described in FASEB J 2002 Nov 16 (13) 1811-3 Epub 2002 Sep 5 and Naunyu-Schmiedeberg's Arch Pharmacol (2001) 364 422-429, respectively. Although oligonucleotides encompassed by the above-described Formula A are described in these references, these oligonucleotides are nothing more than the oligonucleotides that happened to be used in experiments for inhibiting NF-κB, and these references do not disclose or suggest that the binding affinity of the oligonucleotide is increased when the base located upstream of, and adjacently to the consensus sequence is G, and the base located downstream of, and adjacently to the consensus sequence is C.

Although bases are lower-cased in SEQUENCE LISTING, they are upper-cased in the present description and claims. Nucleotides A, G, T, C and U are the nucleotides having adenine, guanine, thymine, cytosine and uracil as the base, respectively, and have the same meanings as a, g, t, c and u in SEQUENCE LISTING, respectively.

In many cases, transcription factors such as NF-κB form families, respectively, and the sequence in the genomic DNA to which the transcription factors belonging to the respective family commonly bind is called "consensus sequence". The term "Consensus Sequence" in the above-described Formula A and in Formula C described below have this meaning. Examples of the transcription factors include, but not limited to, NF-κB, E2F, GATA-3, STAT-1, STAT-6, Ets and AP-1. These transcription factors *per se* are well-known, and the consensus sequence(s) of the respective transcription factor is(are) also well-known.

Among the known transcription factors, a preferred example is NF-κB. "NF-κB" (nuclear factor kappa B) is a collective name of a family of transcription factors (NF-κB/Rel family, for example, P52, P50, P65, cRel, RelB and the like), which have a role in regulating expression of the genes involved in immunoreactions. When NF-κB binds to the binding site in the genomic gene, the genes involved in immunoreactions are overexpressed. Therefore, NF-κB is known to be involved in allergic diseases such as atopic dermatitis and rheumatoid arthritis, and autoimmune diseases, which are caused by immunoreactions, as well as in ischemic diseases such as myocardial infarction and various diseases such as arteriosclerosis.

Examples of the consensus sequences include GGGRHTYYHC (wherein R represents A or G; Y represents C or T; and H represents A, C or T) (SEQ ID NO:1) for NF-κB; TTTSSCGS (wherein S represents G or C) for E2F; WGATAR (wherein W represents A or T; and R represents A or G) for GATA-3; TTCNNNGAA (wherein N represents A, G, T or C) for STAT-1; TTCNNNNGAA (wherein N represents A, G, T or C) (SEQ ID NO:5) for STAT-6; MGGAW (wherein M represents A or C; and W represents A or T) for Ets; and TGASTMA (wherein S represents G or C; and M represents A or C) for AP-1. More specific examples of the consensus sequences include, but not limited to, GGGATTTCCC (SEQ ID NO:2) and GGGACTTTCC (SEQ ID NO:3) for NF-κB; TTTCCCGC for E2F; AGATAG for ATA-3; TCCGGGAA for STAT-1; TTCCCAAGAA (SEQ ID NO:6) for STAT-6; CGGAA for Ets; and TGAGTCA for AP-1.

In the above-described Formula A, m and n are each preferably an integer of not more than 6, more preferably an integer of 2 to 6. In the above-described Formula A, n is preferably an integer of 0, 1, 2 or 3. Although it is generally thought that the flanking sequences of decoy oligonucleotides preferably have a size of 1 to 10 or 1 to 20 bases, the present inventors discovered that there is a tendency that the shorter the 3' flanking sequence within the range of not less than 1 base, the higher the transcription factor-binding activity, and that the longer the 5' flanking sequence (provided that preferably within the range of 2 to 6), the higher the transcription factor-binding activity.

In the above-described Formula A, although the flanking sequences of the sense strand which is a consensus sequence interposed between G at the 5'-side and C at the 3'-side may be arbitrary sequences, the 5' flanking sequence, that is, "N(m)" in Formula A, is one, for example, selected from the group consisting of GA, TGA, TTGA, CTTGA, CCTTGA, ACTTGA, AGTTGA, ACCA, AGCT, AGTATC, AGGC, TTAAC, TTTG and GTCCCAC, preferably one selected from the group consisting of GA, TGA, TTGA, CTTGA and CCTTGA. The 3' flanking sequence, that is, "N(n)" in Formula A is preferably one independently selected from the group consisting of AGGC, TTAAC, TTTG, GTCCCAC, GC, CC, G and C, and especially preferably C or CC. Specific examples of the preferred combination of the flanking sequences include those wherein the 5' flanking sequence and 3' flanking sequence are, respectively, (1) CCTTGA and CC, (2) GA and C or CC, (3) TGA and C or CC, and (4) TTGA and C or CC. The combination of (1) CCTTGA and CC is especially preferred.

Thus, examples of the preferred mode are the double-stranded oligonucleotides composed of a sense strand oligonucleotide and an antisense oligonucleotide complementary thereto, which sense strand oligonucleotide is one wherein, in the above-described Formula A, "N(m)" is CCTTGA and/or wherein the "N(n)" at the 3'-end of the consensus sequence is CC, that is, one comprising a sequence of CCTTGAG-Consensus Sequence-CCC, GAG-Consensus Sequence-CC, GAG-Consensus Sequence-CCC, TGAG-Consensus Sequence-CC, TGAG-Consensus Sequence-CCC, TTGAG-Consensus Sequence-CC or TTGAG-Consensus Sequence-CCC.

The oligonucleotide of the present invention may contain one or more of various modified bases. For example, it may contain a base(s) having a modification(s) such as phosphorothioate, methylphosphonate, phosphorodithioate, phosphoroamidate, boranophosphate, methoxyethyl phosphate, morpholinophosphoroamidate, peptide nucleic acid (PNA), locked nucleic acid (LNA), dinitrophenylation (DNP) and O-methylation. In some cases, although some of these (for example, O-methylation, DNP and the like) are modifications for ribonucleosides, in the present invention, the oligonucleotide may be synthesized such that ribonucleoside(s) is(are) employed for constituting the deoxyribonucleotide (s) to be modified in the oligonucleotide, and the base(s) thereof may be modified. Among the modified oligonucleotides, those having phosphorothioated bases (that is, the bond(s) between nucleosides is(are) phosphorothioate bond(s)) are preferred. All of the bases constituting the oligonucleotide may be modified, or one or more of the bases may be modified. In some cases, it is preferred that the bases constituting the consensus sequence be not modified, or that the bases constituting the consensus sequence be not modified and all of the bases other than those constituting the consensus sequence be modified or only all of the bases constituting the flanking sequences be modified. One of the preferred modes of the present invention is the above-described oligonucleotide wherein at least one internucleoside bond at the 5'-end and/or at least one internucleoside bond at the 3'-end are phosphorothioate bonds. At least one of the internucleoside bonds at the 5'-end and 3'-end, respectively, are preferably phosphorothioate bonds. Especially, two internucleoside bonds at each of the 5'-end and 3'-end are preferably phosphorothioate bonds, that is, in both of the 5'-end and 3'-end, respectively, the bonds between 3 bases from the respective ends are preferably phosphorothioate bonds. Phosphorothioate bond is the bond wherein one of the two non-crosslinking oxygen atoms bound to the phosphorus atom constituting the phosphate bond between adjacent nucleotides is replaced with a sulfur atom. The methods *per se* to phosphorothioate the bond between arbitrary adjacent bases are well-known, and phosphorothioation may be carried out by, for example, the method described in Marina A. et al., The Journal of Biological Chemistry, 1995, Vol. 270, Number 6, pp. 2620-2627. Phosphorothioated oligonucleotides are also commercially synthesized.

By conducting a modification such as phosphorothioation, although the stability of the oligonucleotide in cells is improved, cytotoxicity is thought to be increased. Therefore, it is preferred to limit the number of sites to be phosphorothioated to a small number, and yet to be able to retain the stability in cells. To attain this, the present inventors prepared various oligonucleotides having phosphorothioates at various sites and tried to develop oligonucleotides having sufficient intracellular stability which enables the oligonucleotide to function as a decoy oligonucleotide or antisense oligonucleotide, and having sufficiently reduced cytotoxicity. As a result, the present inventors further discovered that double-stranded decoy oligonucleotides having phosphorothioate bonds in specific patterns have an activity desired for decoy oligonucleotides. Thus, the present invention includes double-stranded decoy oligonucleotides having phosphorothioate bonds in specific patterns.

One of these specific patterns is that at least one phosphorothioate group is contained in 2 or 3 bases from each of the both ends, preferably that the bonds between the 3 bases from each of the both ends are phosphorothioate bonds in at least one of the strands. Another pattern is that in both of the sense and antisense strands, the bonds between the 3 bases from the respective 5'-ends are phosphorothioate bonds.

Other specific patterns mentioned above include the cases wherein in both of the strands, consecutive 2 or 3 phosphorothioate bonds are repeated, and between these repeats, consecutive 2 to 5 phosphodiester bonds are interposed. In the case of this pattern, it is preferred that, except for the base pairs of 2 or 3 bases at the both ends of the double strand, the both strands do not simultaneously have phosphorothioate bonds at the same site, that is, in base pairs of two adjacent bases, the bonds are preferably not simultaneously phosphorothioate bonds. It is also preferred that the oligonucleotide do not have a region in which a phosphorothioate bond is not contained at all, said region being of not less than 7 consecutive base pairs, more preferably not less than 6 consecutive base pairs, still more preferably not less than 5 consecutive base pairs, especially preferably not less than 3 or 4 consecutive base pairs.

More particularly, the oligonucleotides satisfying at least one selected from the following are preferred: (1) In at least one of sense and antisense strands, the bonds between 3 bases at respective one or both ends of the strand are phosphorothioate bonds; (2) In each of the both sense and antisense strands, the bonds between 3 bases at each of both ends contain a phosphorothioate bond(s), but other than these, no adjacent base pairs bonded through a phosphorothioate bond are not contained in any of the strands; (3) In any of the sense and antisense strands, 4 or more consecutive phosphorothioate bonds are not contained; (4) In each of the sense and antisense strands, 2 or 3 consecutive phosphorothioate bonds are contained, (5) In each of the both sense and antisense strands, the bond between the 2 bases at the 5'-end is phosphorothioate bond; (6) In each of the both sense and antisense strands, the bonds between the 3 bases at the 5'-end are phosphorothioate bonds; (7) In each of the both sense and antisense strands, the bond between 2 bases at the 3'-end is a phosphorothioate bond; and (8) In each of the both sense and antisense strands, the bonds between 3 bases at the 3'-end are phosphorothioate bonds.

According to the second aspect, the present invention provides a double-stranded oligonucleotide formed by hybridization of a sense strand oligonucleotide of the following Formula C:

5'-N(x)-Consensus Sequence-N(y)-3'     Formula C

(wherein N(x) is a flanking sequence at the 5'-end, and represents that "N"(s) in the number of x is(are) ligated; N(y) is a flanking sequence at the 3'-end, and represents that "N"(s) in the number of y is(are) ligated; all "N"s each independently represents nucleotide A, G, T, C or U; x and y represent each independently an integer of 1 to 20)
and an antisense strand oligonucleotide complementary to the sense strand oligonucleotide, the antisense strand oligonucleotide comprising consecutive 4 bases wherein the bonds therebetween are phosphorothioate bonds, the consecutive 4 bases not including the base at an end of the oligonucleotide.

In Formula C, the Consensus Sequence and preferred examples thereof are the same as the Consensus Sequence in Formula A described above. In Formula C, x and y are each preferably an integer of not more than 7. Especially, x is preferably an integer of 3 to 7, and y is preferably an integer of 1, 2, 3 or 4.

In Formula C, it is preferred that only one set of the above-described consecutive 4 bases be contained in the antisense strand oligonucleotide, and especially, that only one set of the above-described consecutive 4 bases be contained in the above-described double-stranded oligonucleotide.

It is also included in the phosphorothioation pattern of the present invention that 2 or 3 consecutive phosphorothioate bonds are contained, and each of the both strands does not contain adjacent base pairs bonded through a phosphorothioate bond other than the bonds between the 3 bonds at each of the both ends of each of the both strands. In this pattern, it is preferred that the phosphorothioate bonds be not unevenly distributed to only one of the strands, but preferred that both strands contain 2 or 3 consecutive phosphorothioate bonds.

The sequence represented by the above-described Formula C is preferably encompassed by the above-described Formula A. In other words, it is preferred that the double-stranded oligonucleotide formed by hybridization of the sense strand oligonucleotide represented by the above-described Formula A and the antisense strand oligonucleotide complementary to the sense strand oligonucleotide be simultaneously a double-stranded oligonucleotide formed by hybridization of a sense strand oligonucleotide of the above-described Formula C and the antisense strand oligonucleotide complementary to the sense strand oligonucleotide, the antisense strand oligonucleotide comprising consecutive 4 bases wherein the bonds therebetween are phosphorothioate bonds, the consecutive 4 bases not including the base at an end of the oligonucleotide. In this case, each of the constituents (including m and n) in Formula A as well as their preferred examples and ranges are the same as those described above for Formula A.

Accordingly, preferred examples of the NF-κB decoy oligonucleotide sequences according to the present invention (including both of the first and second aspects of the present invention) include:

the double-stranded oligonucleotide composed of 5'-C*C*TTGAAGG*G*A*TTTCCCT*C*C-3' (SEQ ID NO:7) and its complementary strand 5'-G*G*AGGGAAA*T*C*CCTTCAA*G*G-3',
the double-stranded oligonucleotide composed of 5'-C*C*TTG*A*AGGGATTT*C*CCT*C*C-3' (SEQ ID NO:7) and its complementary strand 5'-G*G*AGGGAAA*T*C*CCTTC*G*G-3',
the double-stranded oligonucleotide composed of 5'-C*C*TTGAAGGGATTTCCCT*C*C-3' (SEQ ID NO:7) and its complementary strand 5'-G*G*AGGGAAA*T*C*CCTTC*G*G-3',
the double-stranded oligonucleotide composed of 5'-C*C*TTG*A*AGGGATTTCCCT*C*C-3' (SEQ ID NO:7) and its complementary strand 5'-G*G*AGGGAAA*T*C*CCTTC*G*G-3',
the double-stranded oligonucleotide composed of 5'-C*C*TTGAAGGGATTT*C*CCT*C*C-3' (SEQ ID NO:7) and its complementary strand 5'-G*G*AGGGAAA*T*C*CCTTC*G*G-3',
the double-stranded oligonucleotide composed of 5'-CCTTG*A*AGGGATTT*C*CCTCC-3' (SEQ ID NO:7) and its complementary strand 5'-GGAGGGAAA*T*C*CCTTCGG-3', the double-stranded oligonucleotide composed of 5'-CCTTG*A*AGGGATTT*C*CCT*C*C-3' (SEQ ID NO:7) and its complementary strand 5'-GGAGGGAAA*T*C*CCTTC*G*G-3', the double-stranded oligonucleotide composed of 5'-C*C*TTG*A*AGGGATTT*C*CCTCC-3' (SEQ ID NO:7) and its complementary strand 5'-G*G*AGGGAAA*T*C*CCTTCGG-3', and the like (the mark "*" indicates a phosphorothioate bond (same as below)). It has been confirmed that the activity of these 8 types of oligonucleotides have higher activity than the decoy composed of the same base sequence in which all of the bases are phosphorothioated. See the Examples below.

Examples of another preferred mode of the NF-κB decoy oligonucleotide sequence include the following:

the double-stranded oligonucleotide composed of 5'-C*C*TTGAGGGGATTTCCCC*C*C-3' (SEQ ID NO:4: having phosphorothioation in part) and its complementary strand 5'-G*G*GGGAAATCCCCTCAA*G*G-3', the double-stranded oligonucleotide composed of 5'-C*C*TTG*A*GGGGATTT*C*CCCCC-3' (SEQ ID NO:4: having phosphorothioation in part) and its complementary strand 5'-G*G*GGGAAA*T*C*CCCTCAAGG-3', the double-stranded oligonucleotide composed of 5'-G*A*GGGGATTT*C*CCC-3' (SEQ ID NO:8) and its complementary strand 5'-GGGGAAA*T*C*CCCTC-3', the double-stranded oligonucleotide composed of 5'-G*A*GGGGATTT*C*CCC-3' (SEQ ID NO:8) and its complementary strand 5'-G*GGGAAA*T*C*CCCTC-3', the double-stranded oligonucleotide composed of 5'-G*A*GGGGATTT*C-CCC-3' (SEQ ID NO:8) and its complementary strand 5'-GG*G*GAAA*T*C*CCCTC-3', the double-stranded oligonucleotide composed of 5'-T*T*G*A*GGGGATTT*C*CCC-3' (SEQ ID NO:9) and its complementary strand 5'-G*G*GGAAA*T*C*CCCTCAA-3', the double-stranded oligonucleotide composed of 5'-T*TT*G*A*GGGGATTT*C*CCC-3' (SEQ ID NO:10) and its complementary strand 5'-G*GGGAAA*T*C*CCCTCAA-3', the double-stranded oligonucleotide composed of 5'-TTG*A*GGGGATTT*C*CCC-3' (SEQ ID NO:9) and its complementary strand 5'-GGGGAAA*T*C*CCCTCAA-3', and the like.

Other examples include:

the double-stranded oligonucleotide composed of 5'-C*C*TTGAAGGGATTTCCCT*C*C-3' (SEQ ID NO:7) and its complementary strand 5'-G*G*AGGGAAATCCCT*T*C*AA*G*G-3', the double-stranded oligonucleotide composed of 5'-C*C*TTGAAGGGATTTCCCT*C*C-3' (SEQ ID NO:7) and its complementary strand 5'-G*G*AGGG*A*A*ATCCCTTCAA*G*G-3', the double-stranded oligonucleotide composed of 5'-C*C*TTGAAGGGACTTTCCT*C*C-3' (SEQ ID NO:11) and its complementary strand 5'-G*G*AGGAAAGTCCC*T*T*CAA*G*G-3', the double-stranded oligonucleotide composed of 5'-C*C*TTGAAGGGACTTTCCT*C*C-3' (SEQ ID NO:11) and its complementary strand 5'-G*G*AGGAAAG*T*C-CCTTCAA*G*G-3', the double-stranded oligonucleotide composed of 5'-C*C*TTGAAGGGACTTTCCT*C*C-3' (SEQ ID NO:11) and its complementary strand 5'-G*G*AGGAA*A*G*TCCCTTCAA*G*G-3', and the like.

The oligonucleotide according to the present invention can be prepared by various methods known in the art. Either chemical synthesis methods or biochemical synthesis methods may be employed. For example, they may be chemically synthesized by the amidite polymerization method and the like.

The phosphorothioated oligonucleotides can also be prepared by known methods. Phosphorothioation can be attained by subjecting the nucleotide at the end ligated by the amidite polymerization to detritylation reaction and oxidation reaction with a sulfur-containing compound.

The double-stranded oligonucleotide of the present invention can be used as a decoy for the transcription factor, that is, as an inhibitor of the transcription factor. Needless to say, the term "transcription factor" herein means the transcription factor which binds to the Consensus Sequence in Formula A or Formula C described above.

Since the above-described transcription factor inhibitors exhibit pharmacological effects resulted from inhibition of the transcription factor, they can be used as pharmaceuticals. The present invention relates to a pharmaceutical composition comprising as an effective ingredient the oligonucleotide according to the present invention. Since the oligonucleotide according to the present invention suppresses gene expression caused by a specific transcription factor or expression of a specific gene, the pharmaceutical compositions are useful for prophylaxis and/or therapy of the diseases involving expression of such genes.

More specifically, the pharmaceutical composition can be used as an agent for prophylaxis, amelioration and/or therapy of ischemic diseases, allergic diseases, inflammatory diseases, autoimmune diseases, metastasis/infiltration of cancers, cachexy, vascular restenosis, acute coronary syndrome, brain ischemia, myocardial infarction, reperfusion hindrance of ischemic diseases, atopic dermatitis, psoriasis vulgaris, contact dermatitis, keloid, decubital ulcer, ulcerative colitis, Crohn's disease, nephropathy, glomerulosclerosis, albuminuria, nephritis, renal failure, rheumatoid arthritis, osteoarthritis, asthma, chronic obstructive pulmonary disease (COPD) or cystic fibrosis (CF), vascular restenosis which occurs after PTCA (percutaneous transluminal coronary angioplasty), PTA (percutaneous transluminal angioplasty), bypass surgery, organ transplantation or surgery of an organ (including those caused by using an artificial blood vessel, catheter or stent, or by vein grafting, and including those caused by a surgical treatment for arteriosclerosis obliterans, aneurysm, aorta dissection, acute coronary syndrome, brain ischemia, Marfan syndrome or plaque rupture). Selection of an appropriate target gene depending on the object may easily be attained by those skilled in the art.

For example, in case of a double-stranded oligonucleotide which contains a binding sequence of NF-κB in the consensus sequence and which functions as a decoy oligonucleotide of NF-κB, it may be used as an agent for prophylaxis, amelioration and/or therapy of any diseases caused by NF-κB, including ischemic diseases, allergic diseases, inflammatory diseases, autoimmune diseases and tumors. The fact that cure and/or delay in progress of these diseases can be attained by decreasing the activity of NF-κB is known to those skilled in the art by various references. Thus, the oligonucleotide of the present invention, when delivered to the cells at the diseased site, acts as a decoy oligonucleotide to bind to NF-κB to inhibit the NF-κB activity, thereby inhibiting the gene expression induced by NF-κB.

The pharmaceutical composition may contain other additive(s) such as stabilizers and diluents, in addition to the oligonucleotide of the present invention. Further, it may contain a pharmaceutically acceptable carrier. Examples of the carrier include physiological saline and physiological phosphate buffer.

Since the pharmaceutical composition is administered by oral administration, parenteral administration, topical administration, intratracheal administration, percutaneous administration (application or the like) or by other external administration, it may be formulated in the form of a solution, suspension, liposomal formulation, capsule, tablet, ointment, cream, lotion, emulsion, nasal drop, spray, formulation for nebulizer, formulation for respirator, or powder, which formulation is suited for the dosage form.

The pharmaceutical composition is especially preferably formulated into a form suited for gene transfer (for example, liposomal formulation derived from a virus such as Sendai virus, retrovirus vector, adenovirus vector or the like, but not limited thereto). Those skilled in the art can easily select an appropriate formulation method.

In case of formulating the oligonucleotide in the form of being encapsulated in Sendai virus envelope vector, which is a preferred mode, it may be helpful to refer Example 8 of JP 2001-286282 A for carrying out the formulation.

The pharmaceutical composition according to the present invention is preferably administered parenterally, although not restricted. Examples of the parenteral administration include intravenous, intraarterial, intratracheal, percutaneous, subcutaneous, intradermal, intramuscular and intraperitoneal administration.

The dose per administration and the number of doses can be appropriately selected and changed depending on various conditions such as the object of the administration, the age and body weight of the patient, symptoms and severity of disease. As for the number of doses and frequency of administration, the composition is preferably administered several times, preferably about 1 to 3 times, at intervals of about 2 to 4 weeks. The number of doses may also be determined monitoring the state of the disease. Usually, 0.1 to 10,000 nmol, preferably 1 to 1000 nmol, still more preferably 10 to 100 nmol of the oligonucleotide may be administered.

The present invention will now be illustrated in the following Examples.
However, the present invention is not limited by the description of these Examples.

Examples

1. Transcription factor-binding Activity Test Using NF-κB Decoy Oligonucleotides

(1) Preparation of Oligonucleotides

(i) Series I Oligonucleotides

As described below, 9 types of oligonucleotides designated SEQ-A, SEQ-B, SEQ-E to J, and SEQ-X were prepared. In addition, 3 types of oligonucleotides having phosphorothioate groups, that is, a fully phosphorothioated oligonucleotide SEQ-x having the same sequence as SEQ-X but all of the nucleotides were phosphorothioated; a partially phosphorothioated oligonucleotide SEQ-X-PS having the same sequence as SEQ-X but 2 bases at each of both ends were phosphorothioated; and a partially phosphorothioated oligonucleotide SEQ-I-PS having the same sequence as SEQ-I but 2 bases at each of both ends were phosphorothioated; were prepared.

SEQ-A AGTTGAGGGGATTTCCCCAGGC (SEQ ID NO:12)
SEQ-B CCTTGAAGGGACTTTCCTCC (SEQ ID NO:11)

SEQ-E AGTATCGGGGATTTCCCCTTAAC (SEQ ID NO:13)
SEQ-F AGCTGGGGATTTCCCCTTTG (SEQ ID NO:14)
SEQ-G ACCAGGGGATTTCCCCGTCCCAC (SEQ ID NO:15)
SEQ-H AGTTGAGGGGATTTCCCCGC (SEQ ID NO:16)
SEQ-I CCTTGAGGGGATTTCCCCCC (SEQ ID NO:4)
SEQ-J ACTTGAAGGGATTTCCCTCC (SEQ ID NO:17)
SEQ-X CCTTGAAGGGATTTCCCTCC (SEQ ID NO:7)
SEQ-x C*C*T*T*G*A*A*G*G*G*A*T*T*T*C*C*C*T*C*C (the one wherein all bases in SEQ ID NO:7 were phosphorothioated)
SEQ-X-PS C*C*TTGAAGGGATTTCCCT*C*C (the one wherein 2 bases at each of both ends of SEQ ID NO:7 were phosphorothioated)
SEQ-I-PS C*C*TTGAGGGGATTTCCCC*C*C (the one wherein 2 bases at each of both ends of SEQ ID NO:4 were phosphorothioated)
The mark "*" indicates a site of a phosphorothioate bond. These sequences may be applied to Formula A as shown in Table 1.

Table 1

| Formula A | 5'-N(m) | G | Consensus Sequence | C | N(n)-3' |
|---|---|---|---|---|---|
| SEQ-A | AGTTGA | G | GGGATTTCCC | C | AGGC |
| SEQ-B | CCTTGA | A | GGGACTTTCC | T | CC |
| SEQ-E | AGTATC | G | GGGATTTCCC | C | TTAAC |
| SEQ-F | AGCT | G | GGGATTTCCC | C | TTTG |
| SEQ-G | ACCA | G | GGGATTTCCC | C | GTCCCAC |
| SEQ-H | AGTTGA | G | GGGATTTCCC | C | GC |
| SEQ-I | CCTTGA | G | GGGATTTCCC | C | CC |
| SEQ-J | ACTTGA | A | GGGATTTCCC | T | CC |
| SEQ-X | CCTTGA | A | GGGATTTCCC | T | CC |
| SEQ-x | C*C*T*T*G*A* | A* | G*G*G*A*T*T*T*C*C*C* | T* | C*C |
| SEQ-X-PS | C*C*TTGA | A | GGGATTTCCC | T* | C*C |
| SEQ-I-PS | C*C*TTGA | G | GGGATTTCCC | C* | C*C |

Each of the oligonucleotides was prepared by the conventional amidite polymerization method (see Polymer Chemistry and Functional Design of Nucleic Acids (The Society of Polymer Science, Japan/Research Group on Polymers and Biosciences eds.) or the like). In the preparation of unmodified oligonucleotides, to a nucleotide immobilized on a column, a sufficient amount of an amidite (the desired nucleotide) was added and polymerization reaction was allowed to occur. After the reaction, detritylation reaction and oxidation reaction with iodine were performed, and the next amidite was added to allow the polymerization reaction. By repeating these operations, each of the oligonucleotides was synthesized. Phosphorothioation was carried out by, after the polymerization reaction of the amidite to be phosphorothioated, detritylation reaction and oxidation reaction with a sulfur-containing chemical substance were performed, and then the next amidite was added for the polymerization reaction.

Further, complementary oligonucleotides each of which is completely complementary to each of these sequences were prepared, and hybridization was carried out by a conventional method to prepare the respective double-stranded oligonucleotides. Here, each of these complementary strands was prepared so as to have phosphorothioate groups in the same pattern. That is, the double-stranded oligonucleotide was prepared such that when one of the bases constituting a complementary base pair in the double-stranded oligonucleotide is phosphorothioated, the other base in this base pair was also phosphorothioated.

(ii) Series II Oligonucleotides

Using the above-described method, Series II oligonucleotides having the same structure as SEQ-x except that the phosphorothioated sites were different. The Series II oligonucleotides are double-stranded oligonucleotides composed of strands complementary to each other, in which the positions of the phosphorothioated bases were different between the complementary strands. The positions of the phosphorothioated bases in each of the oligonucleotides were as shown in the following sequences:

SEQ-1 the double-stranded oligonucleotide composed of 5'-C*C*TTGAAGG*G*A*TTTCCCT*C*C -3' (SEQ ID NO: 7: having phosphorothioate bonds in part) and 5'- G*G*AGGGAAA*T*C*CCTTCAA*G*G -3' (having phosphorothioate bonds in part),

SEQ-2 the double-stranded oligonucleotide composed of 5'-C*C*TTG*A*AGGGATTT*C*CCT*C*C -3' (SEQ ID NO: 7: having phosphorothioate bonds in part) and 5'- G*G*AGGGAAA*T*C*CCTTC*G*G -3' (having phosphorothioate bonds in part),

SEQ-3 the double-stranded oligonucleotide composed of 5'-C*C*TTGAAGGGATTTCCCT*C*C -3' (SEQ ID NO:7: having phosphorothioate bonds in part) and 5'- G*G*AGGGAAA*T*C*CCTTC*G*G -3' (having phosphorothioate bonds in part),

SEQ-4 the double-stranded oligonucleotide composed of 5'-C*C*TTG*A*AGGGATTTCCCT*C*C -3' (SEQ ID NO: 7: having phosphorothioate bonds in part) and 5'- G*G*AGGGAAA*T*C*CCTTC*G*G -3' (having phosphorothioate bonds in part),

SEQ-5 the double-stranded oligonucleotide composed of 5'-C*C*TTGAAGGGATTT*C*CCT*C*C -3' (SEQ ID NO: 7: having phosphorothioate bonds in part) and 5'- G*G*AGGGAAA*T*C*CCTTC*G*G -3' (having phosphorothioate bonds in part),

SEQ-6 the double-stranded oligonucleotide composed of 5'-CCTTG*A*AGGGATTT*C*CCTCC -3' (SEQ ID NO:7: having phosphorothioate bonds in part) and 5'- GGAGGGAAA*T*C*CCTTCGG -3' (having phosphorothioate bonds in part),

SEQ-7 the double-stranded oligonucleotide composed of 5'-CCTTG*A*AGGGATTT*C*CCT*C*C -3' (SEQ ID NO: 7: having phosphorothioate bonds in part) and 5'- GGAGGGAAA*T*C*CCTTC*G*G -3' (having phosphorothioate bonds in part),

SEQ-8 the double-stranded oligonucleotide composed of 5'-C*C*TTG*A*AGGGATTT*C*CCTCC -3' (SEQ ID NO: 7: having phosphorothioate bonds in part) and 5'- G*G*AGGGAAA*T*C*CCTTCGG -3' (having phosphorothioate bonds in part).

The mark "*" indicates a site of a phosphorothioate bond.

After synthesizing each of the strands, double-stranded oligonucleotides were formed and subjected to the binding activity test.

(2) Binding Activity Test

A binding activity test was performed using TransAM NF-κB p65 Transcription Factor Assay Kit (ACTIVE MOTIF).

Complete Lysis Buffer, Complete Binding Buffer, 1 x Wash Buffer and 1 x Antibody Binding Buffer are prepared. Each of the oligonucleotides was dissolved in Complete Binding Buffer to 4 to 7 concentrations within the range of 0.00167 to 167 nmol/L (NF-κB Decoy Oligonucleotide Dilutions). Jurkat nuclear extract was diluted with Complete Lysis Buffer to a concentration of 125 μg protein/mL.

To each of the wells in a plate included in the kit, on which a NF-κB-binding nucleic acid is preliminarily immobilized, 30 μL each of the NF-κB Decoy Oligonucleotide Dilution prepared in (1) described above is added. To the wells of the blank and control, 30 μL each of Complete Binding Buffer was added. Then 20 μL each of the above-described Jurkat nuclear extract was added to each well. To each of the wells of the blank and control, 20 μL each of Complete Lysis Buffer was added. After shaking the plate at room temperature for 1 hour, each well was washed with 200 μL of 1 x Wash Buffer 3 times, and 100 μL each of rabbit anti-NF-κB p65 antibody 1000-fold diluted with 1 x Antibody Binding Buffer was added, followed by shaking the plate at room temperature for 1 hour. Thereafter, each well was washed with 200 μL of 1 x Wash Buffer 3 times, and 100 μL each of HRP-labeled anti-rabbit IgG antibody 1000-fold diluted with 1 x Antibody Binding Buffer was added to each well, followed by shaking the plate at room temperature for 1 hour. Thereafter, each well was washed with 200 μL each of 1 x Wash Buffer 4 times, and 100 μL each of Developing Solution was added, followed by allowing coloring reaction in a dark room for 5 minutes. After the reaction, the coloring reaction was stopped by adding 100 μL each of Stop Solution. Absorbances at 450 nm and 650 nm were measured (from the respective measured values, the corresponding measured value of the blank well was subtracted) by Wallac 1420 ARVOsx Multilabel Counter (PERKIN ELMER), and the ratio of absorbances at 450 nm/650 nm was calculated. The percentage of the absorbance of each NF-κB decoy oligonucleotide at each of the concentrations was calculated taking the ratio of absorbances of the well of control as 100, and $IC_{50}$ of each decoy oligonucleotide was calculated from the approximate curve obtained by plotting the mean values of the above-mentioned percent absorbance.

The $IC_{50}$ of each of the decoy oligonucleotides and the relative $IC_{50}$ of each of the decoy oligonucleotides to the $IC_{50}$ of the decoy oligonucleotide SEQ-x are shown in Tables 2 and 3.

Table 2

| Results of Binding Activity Test of Series I Oligonucleotides | | |
|---|---|---|
| Name | $IC_{50}$ | Relative $IC_{50}$ to that of SEQ-x |
| SEQ-A | 1.51 | 0.38 |
| SEQ-B | 14.15 | 3.56 |
| SEQ-E | 1.91 | 0.48 |
| SEQ-F | 3.4 | 0.86 |
| SEQ-G | 1.71 | 0.43 |
| SEQ-H | 1.08 | 0.27 |
| SEQ-I | 0.384 | 0.10 |
| SEQ-J | 28.17 | 7.09 |
| SEQ-X | 30.41 | 7.66 |
| SEQ-x | 3.972 | 1.00 |
| SEQ-X-PS | 25.23 | 6.35 |
| SEQ-I-PS | 1.06 | 0.27 |

As seen from the above-described results, it was found that the order of the binding activity of the Series I oligonucleotides to NF-κB, from higher to lower, was SEQ-I> SEQ-H > SEQ-A > SEQ-G = SEQ-E > SEQ-F > SEQ-x > SEQ-B > SEQ-J = SEQ-X. Particularly, SEQ-1 had an $IC_{50}$ less than 1/10 that of SEQ-x, and had the highest binding activity.

Each of SEQ-A, SEQ-E, SEQ-F, SEQ-G and SEQ-H had a binding activity higher than that of SEQ-x. Thus, it was observed that the binding activity was increased when G was adjacent to the 5'-end of the consensus sequence and C was adjacent to the 3'-end of the consensus sequence. That is, it is assumed that the binding activity is increased by sandwiching the consensus sequence by G at its 5'-end and C at its 3'-end, or if the same base (G in this case) as the base of the 5'-end of the consensus sequence is adjacent to the 5'-end of the consensus sequence, and the same base (C in this case) as the base of the 3'-end of the consensus sequence is adjacent to the 3'-end of the consensus sequence.

The $IC_{50}$ of SEQ-I-PS in which the 2 bases at each of the 5'-end and 3'-end were phosphorothioated in the sequence of SEQ-I which showed the highest binding activity was less than 1/3 that of SEQ-x, so that the binding activity was much higher than that of SEQ-x. Since the intracellular stability of SEQ-I-PS is thought to be increased by virtue of the phosphorothioation, it is thought that SEQ-I-PS is an especially useful NF-κB decoy oligonucleotide having both high intracellular stability and high binding activity.

Table 3

| Results of Binding Activity Test of Series II Oligonucleotides | | |
|---|---|---|
| Name | $IC_{50}$ | Relative $IC_{50}$ to that of SEQ-x |
| SEQ-x | 2.60 | 1.00 |
| SEQ-1 | 0.84 | 0.32 |
| SEQ-2 | 0.44 | 0.17 |
| SEQ-3 | 2.31 | 0.89 |
| SEQ-4 | 0.75 | 0.29 |
| SEQ-5 | 0.67 | 0.26 |
| SEQ-6 | 1.32 | 0.51 |
| SEQ-7 | 0.32 | 0.12 |
| SEQ-8 | 0.14 | 0.05 |

As seen from the above results, all of the Series II oligonucleotides had higher activities than SEQ-x.

These results suggest that the decoy oligonucleotides in each of which the 2 bases at an end(s) of at least one of the strands are phosphorothioated, and the decoy oligonucleotides each of which has 2 or 3 consecutive phosphorothioated bases, but none of the bases is phosphorothioated in each of all the base pairs other than these bases, have higher transcription factor-binding activity than the fully phosphorothioated decoy oligonucleotides.

(iii) Series III Oligonucleotides

For further study, the decoy oligonucleotides whose sequences are shown in Table 4 below were prepared and binding activity of each of them was determined.
In the base sequences shown in the upper and lower rows described for each oligonucleotide, the upper row shows the base sequence of the sense strand, and the lower row shows the antisense strand complementary to the sense strand. To clearly show that the antisense strand shown in the lower row is complementary to the sense strand shown in the upper row, the antisense strand in the lower row is shown such that its 3'-end is located on the left.
Table 4-1

| Sequence Name | Sequence |
|---|---|
| SEQ-9 | C*C*TTG*A*GGG G A TTT*C*CCC C C (SEQ ID NO:4) |
| | G G AAC T CCC*C*T*AAA G GGG*G*G |
| SEQ-10 | CCUTGAGGGGATTTCCCCCC (SEQ ID NO:18) |
| | GGAACTCCCCTAAAGGGGGG |
| SEQ-11 | CCUUGAGGGGATTTCCCCCC (SEQ ID NO:19) |
| | GGAACUCCCCTAAAGGGGGG |
| SEQ-12 | CCTTGAGGGGATTUCCCCCC (SEQ ID NO:20) |
| | GGAACTCCCCUAAAGGGGGG |
| SEQ-13 | GGGGATTTCCCC (SEQ ID NO:21) |
| | CCCCTAAAGGGG |
| SEQ-14 | AGGGGATTTCCCC (SEQ ID NO:22) |
| | TCCCCTAAAGGGG |
| SEQ-15 | GAGGGGATTTCCCC (SEQ ID NO:8) |
| | CTCCCCTAAAGGGG |
| SEQ-16 | TGAGGGGATTTCCCC (SEQ ID NO:23) |
| | ACTCCCCTAAAGGGG |
| SEQ-17 | TTGAGGGGATTTCCCC (SEQ ID NO:9) |
| | AACTCCCCTAAAGGGG |
| SEQ-18 | CTTGAGGGGATTTCCCC (SEQ ID NO:24) |
| | GAACTCCCCTAAAGGGG |
| SEQ-19 | CCTTGAGGGGATITCCCC (SEQ ID NO:25) |
| | GGAACTCCCCTAAAGGGG |
| SEQ-20 | GGGGATTTCCCCC (SEQ ID NO:26) |
| | CCCCTAAAGGGGG |
| SEQ-21 | AGGGGATTTCCCCC (SEQ ID NO:27) |
| | TCCCCTAAAGGGGG |
| SEQ-22 | GAGGGGATTTCCCCC (SEQ ID NO:28) |
| | CTCCCCTAAAGGGGG |
| SEQ-23 | TGAGGGGATTTCCCCC (SEQ ID NO:29) |
| | ACTCCCCTAAAGGGGG |

(continued)

| Sequence Name | Sequence |
|---|---|
| SEQ-24 | TTGAGGGGATTTCCCCC (SEQ ID NO:30) |
| | AACTCCCCTAAAGGGGG |
| SEQ-25 | CTTGAGGGGATTTCCCCC (SEQ ID NO:31) |
| | GAACTCCCCTAAAGGGGG |
| SEQ-26 | CCTTGAGGGGATTTCCCCC (SEQ ID NO:32) |
| | GGAACTCCCCTAAAGGGGG |

Table 4-2

| Sequence Name | Sequence |
|---|---|
| SEQ-27 | GGGGATTTCCCCCC (SEQ ID NO:33) |
| | CCCCTAAAGGGGGG |
| SEQ-28 | AGGGGATTTCCCCCC (SEQ ID NO:34) |
| | TCCCCTAAAGGGGGG |
| SEQ-29 | GAGGGGATTTCCCCCC (SEQ ID NO:35) |
| | CTCCCCTAAAGGGGGG |
| SEQ-30 | TGAGGGGATTTCCCCCC (SEQ ID NO:36) |
| | ACTCCCCTAAAGGGGGG |
| SEQ-31 | TTGAGGGGATTTCCCCCC (SEQ ID NO:37) |
| | AACTCCCCTAAAGGGGGG |
| SEQ-32 | CTTGAGGGGATTTCCCCCC (SEQ ID NO:38) |
| | GAACTCCCCTAAAGGGGGG |
| SEQ-33 | G*A*GGG G A TTT*C*CCC (SEQ ID NO:8) |
| | C T CCC*C*T*AAA G GGG |
| SEQ-34 | G*A*GGG G A TTT*C*CC C (SEQ ID NO:8) |
| | C T CCC*C*T*AAA G GG*G |
| SEQ-35 | G*A*GGG G A TTT*C*C CC (SEQ ID NO:8) |
| | C T CCC*C*T*AAA G*G*GGG |
| SEQ-36 | T*TG*A*GGG G A TTT*C*CC C (SEQ ID NO:9) |
| | A AC T CCC*C*T*AAA G GG*G |
| SEQ-37 | T*T*G*A*GGG G A TTT*C*C C C (SEQ ID NO:9) |
| | A A C T CCC*C*T*AAA G G*G*G |
| SEQ-38 | TTG*A*GGG G A TTT*C*CCC (SEQ ID NO:9) |
| | AAC T CCC*C*T*AAA G GGG |
| SEQ-39 | CCTTGAGGGGATTTCCC *CCC* (SEQ ID NO:4) |
| | *GGA*ACTCCCCTAAAGGGGGG |
| SEQ-40 | CCTTGAGGGGATTTCCC*CCC* (SEQ ID NO:4) |
| | *GGAACUC*CCCTAAAGGGGGG |

(continued)

| Sequence Name | Sequence |
|---|---|
| SEQ-41 | C-C-TTG-A-GGGG A T TT-C-CCC C C (SEQ ID NO:4) |
| | G G AAC U CCCC-T-A-AA G GGG-G-G |
| SEQ-42 | C-C-T-T-G-A-G-GGGATTTCCC-C-C-C (SEQ ID NO:4) |
| | G-G-A-A-C-U-C-CCCTAAAGGG-G-G-G |
| SEQ-43 | <u>CCT</u>TGAGGGGATTTCCC *CCC* (SEQ ID NO:4) |
| | *GGA*ACTCCCCTAAAGGG <u>GGG</u> |
| SEQ-44 | C*C*TT G A AGGGATTTCCCT*C*C (SEQ ID NO:7) |
| | G*G*AA*C*T*TCCCTAAAGGGA*G*G |
| SEQ-45 | C*C*TTGAAGGGAT T T CCCT*C*C (SEQ ID NO:7) |
| | G*G*AACTTCCCTA*A*A*GGGA*G*G |
| SEQ-46 | C*C*TTGAAGGGACTTTCCT*C*C (SEQ ID NO:11) |
| | G*G*AACTTCCCTGAAAGGA*G*G |

Table 4-3

| Sequence Name | Sequence |
|---|---|
| SEQ-47 | C*C*TTG A A GGGACTTTCCT*C*C (SEQ ID NO:11) |
| | G*G*AAC*T*T*CCCTGAAAGGA*G*G |
| SEQ-48 | C*C*TTGAAGG G A CTTTCCT*C*C (SEQ ID NO:11) |
| | G*G*AACTTCC*C*T*GAAAGGA*G*G |
| SEQ-49 | C*C*TTGAAGGGA C T TTCCT*C*C (SEQ ID NO:11) |
| | G*G*AACTTCCCT*G*A*AAGGA*G*G |
| SEQ-K | AGTTGAAGGGATTTCCCTAGGC (SEQ ID NO:39)<br>TCAACTTCCCTAAAGGGATCCG |
| SEQ-L | CCTTGAGGGGACTTTCCCCC (SEQ ID NO:40)<br>GGAACTCCCCTGAAAGGGGG |
| SEQ-M | ACCAAGGGATTTCCCTGTCCCAC (SEQ ID NO:41)<br>TGGTTCCCTAAAGGGACAGGGTG |
| *: phosphorothioation modification, underline: 2-Ome, bold: LNA, italic: RNA,<br>-: dA Ome | |

First, to investigate the effect of sandwiching the consensus sequence with G at the 5'-end and C at the 3'-end on the binding activity, the binding activities of the decoy oligonucleotides SEQ-A, SEQ-K, SEQ-B, SEQ-L, SEQ-G and SEQ-M in terms of relative $IC_{50}$ to that of the above-described SEQ-x are shown in Table 5 below.

Table 5

| Sequence Name | Relative $IC_{50}$ to SEQ-x |
|---|---|
| SEQ-x | 1.00 |

(continued)

| Sequence Name | Relative $IC_{50}$ to SEQ-x |
|---|---|
| SEQ-A | 0.31 |
| SEQ-K | >1.48 |
| SEQ-B | >1.48 |
| SEQ-L | 0.66 |
| SEQ-G | 0.60 |
| SEQ-M | >1.48 |

As shown in Table 5 above, from comparisons between SEQ-A and SEQ-K; SEQ-L and SEQ-B; and SEQ-G and SEQ-M, respectively, it was found that the binding activity is increased by sandwiching the consensus sequence with G at the 5'-end and C at the 3'-end.

As for the effects by the type of modifying group on the binding activity, as shown in the lines of SEQ-9 to 12, the binding activity was low with any of 2-Ome, LNA and dA Ome. The phosphorothioation (phosphorothioation modification) alone was able to increase the binding activity. Therefore, the relationship between position/number and binding activity was investigated only for phosphorothioation.

Table 6

| Sequence Name | Relative $IC_{50}$ to SEQ-x |
|---|---|
| SEQ-x | 1.00 |
| SEQ-9 | 0.02 |
| SEQ-10 | 1.72 |
| SEQ-11 | >4.18 |
| SEQ-12 | >4.18 |
| SEQ-39 | >2.93 |
| SEQ-40 | >2.93 |
| SEQ-41 | >2.93 |
| SEQ-42 | >2.93 |
| SEQ-43 | >2.93 |
| SEQ-X-PS | >1.67 |
| SEQ-44 | >1.67 |
| SEQ-45 | 1.43 |
| SEQ-46 | >1.67 |
| SEQ-47 | 0.72 |
| SEQ-48 | 0.20 |
| SEQ-49 | 0.19 |

SEQ-9 is a partially phosphorothioated oligonucleotide corresponding to the combination of the sequence of SEQ-I and the phosphorothioated sites in SEQ-8. The binding activities of SEQ-I and SEQ-8 were higher than that of SEQ-x. The binding activity of SEQ-9 corresponding to the combination of the sequence of SEQ-I and the phosphorothioated sites in SEQ-8 was about 50 times higher than that of SEQ-x. Each of SEQ-X-PS and SEQ-46 has phosphorothioation modification at consecutive two sites at each of both ends of both strands. Each of SEQ-44, SEQ-45, SEQ-47, SEQ-48 and SEQ-49 has phosphorothioation modification at consecutive 2 sites at each of both ends of both strands, and at consecutive 3 sites in other region. Each of SEQ-48 and SEQ-49 has phosphorothioation modification at consecutive 3 sites in the consensus sequence, in addition to the modification sites in SEQ-X-PS. The binding activity of SEQ-47 was about not less than 2.5 times higher than that of SEQ-46, and the binding activities of SEQ-48 and SEQ-49 were about not less than 8.5 times higher than that of SEQ-46. From these results, it is seen that binding activity is increased by the fact that the oligonucleotide has phosphorothioation modification at consecutive 2 sites at each of both ends of both strands, or that the oligonucleotide has phosphorothioation modification at consecutive 3 sites in a region other than the two sites at the both ends.

The relative activities of SEQ-X-PS, SEQ-44 and SEQ-45 were further investigated in detail to obtain the results shown in Table 7 below. As expected, the binding activities of both of SEQ-44 and SEQ-45 were higher than that of SEQ-X-PS.

Table 7

| Sequence Name | Relative $IC_{50}$ to SEQ-X-PS |
|---|---|
| SEQ-X-PS | 1.00 |
| SEQ-44 | 0.60 |
| SEQ-45 | 0.16 |

The length of decoy oligonucleotides was then investigated. The results in terms of the relative activity to SEQ-1 are shown in Table 8 below.

Table 8

| Sequence Name | Relative $IC_{50}$ to SEQ-I |
|---|---|
| SEQ-I | 1.00 |
| SEQ-13 | >2.87 |
| SEQ-14 | 1.39 |
| SEQ-15 | 0.61 |
| SEQ-16 | 0.42 |
| SEQ-17 | 0.30 |
| SEQ-18 | 0.28 |
| SEQ-19 | 0.26 |
| SEQ-20 | >3.45 |
| SEQ-21 | 2.07 |
| SEQ-22 | 1.09 |
| SEQ-23 | 0.69 |
| SEQ-24 | 0.52 |
| SEQ-25 | 0.41 |
| SEQ-26 | 0.43 |
| SEQ-27 | >2.2 |
| SEQ-28 | >2.2 |
| SEQ-29 | 2.17 |
| SEQ-30 | 1.24 |
| SEQ-31 | 0.84 |
| SEQ-32 | 0.76 |

The binding activities of SEQ-15, SEQ-16, SEQ-17, SEQ-18, SEQ-19, SEQ-23, SEQ-24, SEQ-25 and SEQ-26 were higher than that of SEQ-1, so that it was found that the shorter the 3' flanking sequence of the sense strand, the higher the binding activity, and that the longer the 5' flanking sequence, the higher the binding activity. From these results, it is thought that the binding activity is increased by the fact that the 3' flanking sequence has 1 or 2 bases and the 5' flanking sequence has not less than 3 bases.

Further, the binding activities of partially phosphorothioated oligonucleotides SEQ-33, SEQ-34, SEQ-35, SEQ-36, SEQ-37 and SEQ-38 having a size of 14 bases or 16 bases were about 50 to about 100 times higher than that of SEQ-x. The sequences used in this study and the relative $IC_{50}$ values to SEQ-x are shown in Table 9 below.

Table 9

| Sequence Name | Relative $IC_{50}$ to SEQ-x |
|---|---|
| SEQ-x | 1.00 |
| SEQ-33 | 0.021 |
| SEQ-34 | 0.014 |
| SEQ-35 | 0.015 |
| SEQ-36 | 0.012 |
| SEQ-37 | 0.015 |
| SEQ-38 | 0.011 |

However, as described above (Table 2), the binding activity of SEQ-8 was about 10 times higher than that of SEQ-6. From these results, it is thought that it is necessary to give phosphorothioation modification at consecutive 2 sites from each of both ends of both strands in cases where the oligonucleotide has a size of 20 bases, but in cases where the oligonucleotide has a size of 14 bases or 16 bases, it is not necessary to give phosphorothioation modification at consecutive 2 sites at each of both ends, but the binding activity is increased only by the phosphorothioation at the same sites as in SEQ-6.

3. Intracellular NF-κB Binding Test

(1) Cells

RAW264.7 cells grown to about 80% confluency in a culture dish were washed with Dulbecco's modified PBS (D-PBS) after removing the culture medium from the dish, and the cells were collected by peeling the cells off from the dish with a scraper after adding an appropriate amount of 10% FBS-containing RPMI1640 medium. After counting the cells, the cells are plated in a 6-well plate (Corning) at a cell density of 6.0 x $10^5$ cells/well/2 mL, and cultured under 5% $CO_2$ in an incubator at 37˚C for 24 hours.

(2) Constitution of Experimental Groups

The experimental groups were as follows:

Group 1: Treated with 2.0 μmol/L of NF-κB ss and not stimulated (n = 3)
Group 2: Treated with 2.0 μmol/L of NF-κB ss and stimulated with LPS (n = 3)
Group 3: Treated with 0.1 μmol/L of SEQ-x and stimulated with LPS (n = 3)
Group: 4 Treated with 0.3 μmol/L of SEQ-x and stimulated with LPS (n = 3)
Group 5: Treated with 0.1 μmol/L of SEQ-I-PS and stimulated with LPS (n = 3)
Group 6: Treated with 0.3 μmol/L of SEQ-I-PS and stimulated with LPS (n = 3)

(3) Addition of NF-κB Decoy Oligonucleotides and NF-κB ss

The cells prepared as described above are washed with D-PBS, and 1 mL of RPMI1640 medium is added to each well. DMRIE-C (1.2-dimyristyloxypropyl-3-dimethyl-hydroxy ethyl ammonium bromide) at a concentration of 2 mg/mL was diluted with RPMI1640 to a concentration of 0.11 mg/mL (DMRIE-C dilution). SEQ-x and SEQ-I-PS were diluted with RPMI1640 to concentrations of 0.2 μmol/L and 0.6 μmol/L (oligonucleotide dilutions). NF-κB ss (single-strand of SEQ ID NO:7) was diluted with RPMI1640 to a concentration of 0.6 μmol/L (NF-κB ss dilution).

Equivolume of DMRIE-C dilution and each oligonucleotide dilution or NF-κB ss were gently mixed and the resulting mixture was left to stand at room temperature for 30 minutes (Mixed Solution). To each well, 1000 μL of the Mixed Solution was added and left to stand in a $CO_2$ incubator for 4 hours. Four hours later, LPS was added to a final concentration of 100 ng/mL and the resulting mixture was left to stand in a $CO_2$ incubator for 1 hour. After the reaction, the cells in the dish were washed twice with iced PBS and collected with a scraper.

(4) Preparation of Nuclear Extract

1 x hypotonic buffer and Complete Lysis Buffer were prepared, and the collected cells were suspended in 100 μL of the 1 x hypotonic buffer. To this suspension, 5 μL of a detergent solution (Detergent) was added, and the resulting mixture was mixed with Vortex for 10 seconds, followed by centrifugation of the resulting mixture at 14,000 x g for 30 seconds. After removing the supernatant, 20 μL of Complete Lysis Buffer was added to the pellet, and the resultant was left to stand at 4˚C for 30 minutes. The resulting mixture was then mixed with Vortex for 30 seconds, and the resultant was centrifuged at 14,000 x g for 10 minutes. The supernatant was recovered in a centrifuge tube and stored at -80˚C. All of the operations are carried out at 4˚C or on ice whenever possible.

(5) NF-κB p65 ELISA

The above-described nuclear extract solution is dissolved immediate before use on ice. The nuclear extract solution is 5-fold diluted with Complete Lysis Buffer. To a 96-well plate included in an NF-κB p65 ELISA kit, 30 μL of Complete Binding Buffer and 20 μL of each of the diluted sample solutions described above were added, and the plate was shaken at room temperature for 1 hour. Then each well was washed 3 times with 200 μL of 1 x Wash Buffer, and 100 μL of HRP-labeled anti-rabbit IgG antibody 1000-fold diluted with 1 x Antibody Binding Buffer was added. Each well was then washed 4 times with 200 μL of 1 x Wash Buffer, and 100 μL of Developing Solution was added, followed by allowing the coloring reaction in a dark room for 5 minutes. After the reaction, 100 μL each of Stop Solution was added to stop the coloring reaction. Absorbances at 450 nm and 650 nm were measured (from the respective measured value, the corresponding measured value of the blank well was subtracted) by Wallac 1420 ARVOsx Multilabel Counter (PERKIN ELMER), and the ratio of absorbances at 450 nm/650 nm was calculated.

Calculation was carried out using Microsoft (trademark) Excel 2002 (Microsoft) was used. The mean values were calculated by using a function AVERAGE of Excel and the standard deviations (S.D.) were calculated by using a function STDEV. The results are shown in Table 10 and Fig. 1 in terms of mean ± standard deviation of respective 3 cases.

Table 10

| | Decoy (μmol/L) | Measured Value | Standard Deviation |
|---|---|---|---|
| | NF-κB ss 1.0 | 0.18 | 0.03 |
| DMRIE-C + Decoy + LPS 100 ng/mL | NF-κB ss 1.0 | 0.95 | 0.03 |
| | SEQ-x 0.1 | 1.07 | 0.05 |
| | SEQ-x 0.3 | 0.14 | 0.15 |
| | SEQ-I-PS 0.1 | 0.04 | 0.02 |
| | SEQ-I-PS 0.3 | 0.00 | 0.00 |

The result of SEQ-x at a concentration of 0.1 μmol/L was almost the same as that of the NF-κB ss LPS-stimulated group which was a control. However, at a concentration of 0.3 μmol/L, SEQ-x exhibited a significant antagonistic effect to NF-κB protein (inhibition ratio: about 85%). SEQ-I-PS exhibited significant antagonistic effect to NF-κB protein at 0.1 μmol/L and 0.3 μmol/L (inhibition ratio: about 99%). Thus, since SEQ-x exhibits antagonistic effect to activated NF-κB protein at 0.3 μmol/L, while SEQ-I-PS exhibits antagonistic effect to activated NF-κB protein at 0.1 μmol/L, it is thought that SEQ-I-PS has a binding activity not less than 3 times higher than SEQ-x.

4. Toxicity Test

(1) Plating to 96-well Plate

Cells grown to about 80% confluency in a culture dish were washed with Dulbecco's modified PBS (D-PBS) after removing the culture medium from the dish, and the cells were detached from the dish by adding an appropriate amount of trypsin-EDTA and leaving the cells to stand at 37˚C in a 5% $CO_2$ incubator for 3 minutes, followed by collecting the cells by adding 10% FBS-containing MEM medium. After centrifugation (1000 rpm, 5 minutes, 25˚C) with a refrigerated centrifuge (Beckman), the supernatant was removed and the cell pellet was re-suspended in 10% FBS-containing MEM medium. After counting the cells, the cells were plated in a 6-well plate (Corning) at a cell density of 5.0 x$10^3$ cells/well/ 50 μL.

(2) Constitution of Experimental Groups

Group 1: Untreated group (n = 3)
Group 2: Group treated with 1.0 μmol/L of SEQ-x (n = 3)
Group 3: Group treated with 3.0 μmol/L of SEQ-x (n = 3)
Group 4: Group treated with 10 μmol/L of SEQ-x (n = 3)
Group 5: Group treated with 30 μmol/L of SEQ-x (n = 3)
Group 6: Group treated with 1.0 μmol/L of SEQ-X (n = 3)
Group 7: Group treated with 3.0 μmol/L of SEQ-X (n = 3)
Group 8: Group treated with 10 μmol/L of SEQ-X (n = 3)
Group 9: Group treated with 30 μmol/L of SEQ-X (n = 3)
Group 10: Group treated with 1.0 μmol/L of SEQ-X-PS (n = 3)
Group 11: Group treated with 3.0 μmol/L of SEQ-X-PS (n = 3)
Group 12: Group treated with 10 μmol/L of SEQ-X-PS (n = 3)
Group 13: Group treated with 30 μmol/E of SEQ-X-PS (n = 3)
Group 14: Group treated with 1.0 μmol/L of SEQ-I-PS (n = 3)
Group 15: Group treated with 3.0 μmol/L of SEQ-I-PS (n = 3)
Group 16: Group treated with 10 μmol/L of SEQ-I-PS (n = 3)
Group 17: Group treated with 30 μmol/L of SEQ-I-PS (n = 3)

(3) Measurement of Toxicity

After Plating the cells, each of the oligonucleotides formulated with 10% FBS-containing RPMI medium to a concentration of 2, 6, 20 and 60 μmol/L, respectively, was added to each well in an amount of 50 μL/well, and the plate was left to stand in a $CO_2$ incubator for 24 hours. Twenty four hours later, 10 μL of WST-1 was added and the plate was left to stand in a $CO_2$ incubator for 30 minutes to 1 hour. After the reaction, absorbances (450 nm/640 nm) were measured with Wallac 1420 ARVOsx (PERKIN ELMER JAPAN).
First, the results of SEQ-X, SEQ-X-PS, SEQ-x and SEQ-I-PS among the Series I decoy oligonucleotides are shown in Fig. 2.
In the measurement at 24 hours later, in the groups in which 1,3, 10 and 30 μmol/L of SEQ-X were added, respectively, enhancement of cell death was not observed. In the groups in which 1, 3 and 10 μmol/L of SEQ-x were added, respectively, enhancement of cell death was not observed. However, in the group in which 30 μmol/L of SEQ-x was added, death of about 25% of the cells was observed. In the groups in which 1, 3 and 10 μmol/L of SEQ-X-PS and SEQ-I-PS were added, respectively, enhancement of cell death was not observed. However, in the group in which 30 μmol/L of SEQ-X-PS and SEQ-1-PS were added, respectively, death of about 10% of the cells was observed. From these results, it is thought that cytotoxicity was reduced by partial phosphorothioation.
Results of the measurements of toxicity for SEQ-I, SEQ-9, SEQ-33, SEQ-34, SEQ-35, SEQ-36, SEQ-37 and SEQ-38 in terms of $LD_{50}$ are shown in Table 11 below.
Table 11

| Sequence Name | $LD_{50}$ (μmol/L) | SD |
|---|---|---|
| SEQ-x | 53.1 | 15.5 |
| SEQ-I | 93.1 | 3.4 |
| SEQ-9 | 80.9 | 5.1 |
| SEQ-33 | 139.8 | 22.7 |

(continued)

| Sequence Name | $LD_{50}$ (μmol/L) | SD |
|---|---|---|
| SEQ-34 | 175.5 | 22.1 |
| SEQ-35 | 119.9 | 15.2 |
| SEQ-36 | 87.8 | 4.4 |
| SEQ-37 | 108.4 | 11.2 |
| SEQ-38 | 84.2 | 4.3 |

As shown in Table 11, the $LD_{50}$ value (median lethal dose) of SEQ-1 was about 1.8 times higher than that of SEQ-x. The $LD_{50}$ values of SEQ-9, SEQ-33, SEQ-34, SEQ-35, SEQ-36, SEQ-37 and SEQ-38 which are partially phosphorothioated oligonucleotides were about 1.5 to about 3 times higher than that of SEQ-x. Therefore, the toxicities of SEQ-9, SEQ-33, SEQ-34, SEQ-35, SEQ-36, SEQ-37 and SEQ-38 were reduced compared with that of SEQ-x. From the comparison between the results of SEQ-x, SEQ-I and SEQ-9, which have a size of 20 bases, it was found that the toxicity was decreased by decreasing the number of phosphorothioated sites.

5. Stability Test

Sample solutions were prepared so as to attain a final concentration of each oligonucleotide sequence of 100 ng/μL, and final concentrations of mouse blood plasma of 10%, 15% and 20%, respectively, and they were incubated at 37°C for 24 hours. Then each of the sample solutions was subjected to separation by 20% denatured polyacrylamide gel electrophoresis, and the resulting bands were stained with ethidium bromide, followed by image analysis with a meter (BIO-RAD). The results of the electrophoresis are shown in Fig. 3.

Experimental Groups

Group 1: SEQ-x + PBS
Group 2: SEQ-x + 10% mouse blood plasma
Group 3: SEQ-x+ 15% mouse blood plasma
Group 4: SEQ-x+ 20% mouse blood plasma
Group 5: SEQ-I + PBS
Group 6: SEQ-I + 10% mouse blood plasma
Group 7: SEQ-I + 15% mouse blood plasma
Group 8: SEQ-I + 20% mouse blood plasma
Group 9: SEQ-I-PS + PBS
Group 10: SEQ-I-PS + 10% mouse blood plasma
Group 11: SEQ-I-PS + 15% mouse blood plasma
Group 12: SEQ-I-PS + 20% mouse blood plasma

SEQ-x was stable in 10%, 15% and 20% mouse blood plasma, respectively. On the other hand, decomposition of SEQ-I in 10%, 15% and 20% mouse blood plasma, respectively, was observed, and in 20% mouse blood plasma, it was almost completely decomposed. On the other hand, SEQ-I-PS was stable in 10% and 15% mouse blood plasma, respectively. Although slight decomposition of SEQ-I-PS was observed in 20% mouse blood plasma, SEQ-I-PS is thought to be significantly more stable than SEQ-I. From these results, it is thought that by phosphorothioating the 2 bases at each of both ends, stability can be significantly increased.

6. Reporter Assay

The reporter assay herein used was the system in which the transcription of a reporter gene introduced into the cells is activated depending on the activation of NF-κB, and the activity of the expressed enzyme is measured. This evaluation system is used by those skilled in the art in the studies of inhibitors of signal transduction.

3.1 Testing Method

(1) Plating Cells to 24-well Plate

Cells were observed to confirm that abnormality was not confirmed and the cells were about 80% confluency. After washing the cells with an appropriate amount of D-PBS(-), an appropriate amount of Trypsin-EDTA was added, and the cells were allowed to react at 37˚C in a 5% $CO_2$ incubator for 3 minutes, thereby detaching the cells. The detached cells were suspended in 10% FBS-containing MEM medium and recovered in a centrifuge tube, followed by centrifugation (1000 rpm, 5 minutes, 25˚C) with a refrigerated centrifuge (Beckman) and aspiration of the supernatant. The sedimented cells were re-suspended in 10% FBS-containing MEM medium, and the cells were counted with an improved hemocytometer. The cells were plated in the wells of a 24-well plate (Corning) in the number of 4.0 x $10^4$ cells/well/0.5 mL, and incubated at 37˚C in a 5% $CO_2$ incubator for 24 hours.

(2) Constitution of Experimental Groups

Experiment 1 and Experiment 2 were carried out independently. Experimental groups in each experiment were as follows:

<u>Experiment 1</u>

1. Unstimulated Group: Treated with FuGENE6 + pNF (pNFκB-SEAP plasmid) for 4 hours → no stimulation
2. TNF-α-stimulated Group: Treated with FuGENE6 + pNF for 4 hours → stimulated with TNF-α for 24 hours
3. SEQ-x Group: Treated with FuGF-NE6 + pNF +SEQ-x (0.1 μmol/L) for 4 hours
4. SEQ-9 Group: Treated with FuGENE6 + pNF +SEQ-9 (0.1 μmol/L) for 4 hours → stimulated with TNF-α for 24 hours
5. SEQ-33 Group: Treated with FuGENE6 + pNF +SEQ-33 (0.1 μmol/L) for 4 hours → stimulated with TNF-α for 24 hours
6. SEQ-34 Group: Treated with FuGENE6 + pNF +SEQ-34 (0.1 μmol/L) for 4 hours → stimulated with TNF-α for 24 hours
7. SEQ-35 Group: Treated with FuGENE6 + pNF +SEQ-35 (0.1 μmol/L) for 4 hours → stimulated with TNF-α for 24 hours

<u>Experiment 2</u>

1. Unstimulated Group: Treated with FuGENE6 + pNF for 4 hours → no stimulation
2. TNF-α-stimulated Group: Treated with FuGENE6 + pNF for 4 hours → stimulated with TNF-α for 24 hours
3. SEQ-x Group: Treated with FuGENE6 + pNF +SEQ-x (0.1 μmol/L) for 4 hours → stimulated with TNF-α for 24 hours
4. SEQ-36 Group: Treated with FuGENE6 + pNF +SEQ-36 (0.1 μmol/L) for 4 hours → stimulated with TNF-α for 24 hours
5. SEQ-37 Group: Treated with FuGENE6 + pNF +SEQ-37 (0.1 μmol/L) for 4 hours → stimulated with TNF-α for 24 hours
6. SEQ-38 Group: Treated with FuGENE6 + pNF +SEQ-38 (0.1 μmol/L) for 4 hours → stimulated with TNF-α for 24 hours

In each experimental group, n = 3.

(3) Addition of Each Oligonucleotide

HeLa cells were plated in a 24-well plate, and cultured for 24 hours. The cells were washed with D-PBS(-) and 250 μL of MEM medium was added. FuGENE6 was 83.3-fold diluted with MEM medium (FuGENE6 dilution). DNA mixed solutions having the following compositions were prepared.

Experiment 1

Table 12

| Group Number | Each Oligonucleotide | pNF |
|---|---|---|
| 1, 2 | - | 4.0 ng/μL |
| 3-8 | 0.4 μmol/L | 4.0 ng/μL |

Experiment 2

Table 13

| Group Number | Each Oligonucleotide | pNF |
|---|---|---|
| 1, 2 | - | 4.0 ng/μL |
| 3-6 | 0.4 μmol/L | 4.0 ng/μL |

Equivolume of the thus prepared each of the DNA mixed solutions and FuGENE6 dilution were gently mixed and allowed to react at room temperature for 30 minutes (FuGENE6 mixed solution). After the reaction, 250 μL each of the each FuGENE6 mixed solution was added to a 24-well plate and allowed to react for 4 hours. After washing each of the wells with D-PBS(-), 500 μL of 10% FBS-containing MEM medium was added, and the resulting mixture was allowed to react for about 20 hours. After the reaction, each well was washed with D-PBS(-) and 500 μL of MEM medium was added. TNA-α was added to a final concentration of 50 ng/mL and the resulting mixture was allowed to react for about 24 hours. After the reaction, the culture supernatant was recovered in a 1.5 mL tube, and centrifuged at 10,000 rpm for 5 minutes at 4˚C with a micro high speed refrigerated centrifuge. The supernatant alone was transferred to another 1.5 mL tube, and stored in a freezer (about -20˚C) for cell culture chamber.

(4) Measurement of Reporter

5 x Dilution Buffer was 5-fold diluted with distilled water to prepare 1 x Dilution Buffer. Further, 25 mmol/L of CSPD Substrate was 20-fold diluted with Chemiluminescent Enhancer to prepare 1.25 mmol/L of CSPD Substrate.

Chemiluminescent Enhancer, Assay Dilution and the prepared 1 x Dilution Buffer were restored to room temperature. To a 1.5 mL tube, 15 μL each of the samples was added. Then 45 μL of 1 x Dilution Buffer was added, and the resultant was mixed by tapping. The mixture was allowed to react at 65˚C for 30 minutes. After the reaction, the mixture was left to stand on ice for 3 minutes, and the 1.5 mL tube was restored to room temperature. Then 60 μL of Assay Dilution was added, and the resultant was mixed by tapping, followed by allowing the mixture to react at room temperature for 5 minutes. After the reaction, 60 μL of 1.25 mmol/L of CSPD Substrate was added, and the resultant was mixed by tapping, followed by allowing the mixture to react at room temperature for 10 minutes. After the reaction, the reaction mixture was transferred to a 96-well plate, and relative luminescence intensity (RLU) was measured with Wallac 1420 ARVOsx Multilabel Counter (PERKIN ELMER). In this test, the concentration of each oligonucleotide sequence was 0.1 μmol/L.

In the above-described Experiment 1, the RLU value of the TNF-α group was about 80 times higher than that of the unstimulated group. The RLU value of the SEQ-x group was about the same as that of the TNF-α group. The RLU values of SEQ-9, SEQ-33, SEQ-34 and SEQ-35 groups were lower than that of the TNF-α group, and inhibition ratio of SEQ-9 group was about 70%, that of SEQ-33 was about 70%, that of SEQ-34 was about 99% and that of SEQ-35 was about 70%.

In Experiment 2, the RLU value of the TNF-α group was about 145 times higher than that of the unstimulated group. The RLU value of the SEQ-x group was about the same as that of the TNF-α group. The RLU values of SEQ-36, SEQ-37, and SEQ-38 groups were lower than that of the TNF-α group, and inhibition ratio of SEQ-36 group was about 80%, that of SEQ-37 was about 75%, and that of SEQ-38 was about 90%.

Experimental Group 1

Table 14

| Experimental Group | RLU | SD |
|---|---|---|
| Unstimulated | 740 | 191 |
| TNF-α | 57684 | 20706 |
| 0.1 μmol/L SEQ-x | 69473 | 23268 |
| 0.1 μmol/L SEQ-9 | 17092 | 2139 |
| 0.1 μmol/L SEQ-33 | 18535 | 1847 |
| 0.1 μmol/L SEQ-34 | 1033 | 380 |
| 0.1 μmol/L SEQ-35 | 15343 | 3121 |

Experimental Group 2

Table 15

| Experimental Group | RLU | SD |
|---|---|---|
| Unstimulated | 243 | 34 |
| TNF-α | 35373 | 21377 |
| 0.1 μmol/L SEQ-x | 28204 | 16461 |
| 0.1 μmol/L SEQ-36 | 6080 | 2474 |
| 0.1 μmol/L SEQ-37 | 9044 | 4382 |
| 0.1 μmol/L SEQ-38 | 4495 | 2952 |

SEQ-x group did not show inhibition action against activation of transcription. SEQ-9, SEQ-33, SEQ-34, SEQ-35, SEQ-36, SEQ-37 and SEQ-38 groups inhibited activation of transcription by about 70% to about 99%. From these results, it is thought that SEQ-9, SEQ-33, SEQ-34, SEQ-35, SEQ-36, SEQ-37 and SEQ-38 inhibit NF-κB signal-dependent activation of transcription of the gene at a lower concentration than SEQ-x.

## Claims

1. A double-stranded oligonucleotide formed by hybridization of a sense strand oligonucleotide of the following Formula A:

$$5'\text{-N(m)-G-Consensus Sequence -C-N(n)-}3' \qquad \text{(Formula A)}$$

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; and the Consensus Sequence represents a consensus sequence to which a transcription factor binds) and an antisense strand oligonucleotide complementary to said sense strand oligonucleotide (excluding those oligonucleotides wherein the sense strand oligonucleotide thereof is the sequence AGTTGAGGGGACTTTCCCAGGC (SEQ ID NO:42) or GATCGAGGGGACTTTCCCTAG (SEQ ID NO:43)).

2. The oligonucleotide according to claim 1, wherein said m and n are integers of not more than 6.

3. The oligonucleotide according to claim 2, wherein in said Formula A, said m is an integer of 2 to 6.

4. The oligonucleotide according to claim 2 or 3, wherein in said Formula A, n is an integer of 0, 1, 2 or 3.

**5.** A double-stranded oligonucleotide formed by hybridization of a sense strand oligonucleotide of the following Formula A:

$$5'\text{-N(m)-G-Consensus Sequence -C-N(n)-3'} \qquad \text{(Formula A)}$$

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; Consensus Sequence means the consensus sequence to which a transcription factor binds; and the N(m) is a sequence selected from the group consisting of GA, TGA, TTGA, CTTGA and CCTTGA) and an antisense strand oligonucleotide complementary to said sense strand oligonucleotide.

**6.** The oligonucleotide according to claim 4 or 5, wherein in said Formula A, N(n) is C or CC.

**7.** The oligonucleotide according to claim 6, wherein in said Formula A, N(m) is an oligonucleotide whose sequence is CCTTGA, and N(n) is an oligonucleotide whose sequence is CC.

**8.** The oligonucleotide according to any one of claims 1 to 7, wherein bonds between bases comprise a phosphorothioate bond.

**9.** The oligonucleotide according to claim 8, wherein 3 bases at an end(s) of at least one of said sense strand and antisense strand are bound through phosphorothioate bonds.

**10.** The oligonucleotide according to claim 8, wherein 3 bases each at both ends of said sense strand and said antisense strand comprise a phosphorothioate bond, but other than these, none of said strands comprises adjacent base pairs bound through a phosphorothioate bond.

**11.** The oligonucleotide according to any one of claims 8 to 10, wherein 4 or more consecutive phosphorothioate bonds do not exist in any of said sense strand and said antisense strand.

**12.** The oligonucleotide according to any one of claims 8 to 11, wherein 2 or 3 consecutive phosphorothioate bonds exist(s) in each of said sense strand and said antisense strand.

**13.** The oligonucleotide according to any one of claims 8 to 12, wherein the bond between the two bases at the 5'-end is a phosphorothioate bond in both of said sense strand and said antisense strand, respectively.

**14.** The oligonucleotide according to claim 13, wherein the bonds between 3 bases at the 5'-end are phosphorothioate bonds in both of said sense strand and said antisense strand, respectively.

**15.** The oligonucleotide according to any one of claims 8 to 14, wherein the bond between 2 bases at the 3'-end is a phosphorothioate bond in both of said sense strand and said antisense strand, respectively.

**16.** The oligonucleotide according to claim 15, further **characterized in that** the bonds between 3 bases at the 3'-end are phosphorothioate bonds in both of said sense strand and said antisense strand, respectively.

**17.** The oligonucleotide according to any one of claims 1 to 16, wherein said consensus sequence is a binding sequence of NF-κB, E2F, GATA-3, STAT-1, STAT-6, Ets or AP-1.

**18.** The oligonucleotide according to claim 17, wherein said consensus sequence is a binding sequence of NF-κB, said consensus sequence being represented by GGGRHTYYHC (wherein R represents A or G; Y represents C or T; H represents A, C or T) (SEQ ID NO:1).

**19.** The oligonucleotide according to claim 18, wherein said binding sequence of NF-κB is GGGATTTCCC (SEQ ID NO:2) or GGGACTTTCC (SEQ ID NO:3).

**20.** The oligonucleotide according to claim 20, wherein said sense strand oligonucleotide has a sequence CCTTGAGGGGATTTCCCCCC (SEQ ID NO:4).

**21.** The oligonucleotide according to claim 20, wherein among the bonds between bases in said sense strand CCTTGAGGGGATTTCCCCCC (SEQ ID NO:4), only the first, second, 5th, 6th, 14th and 15th bonds from the 5'-end are phosphorothioate bonds, and among the bonds between bases in said antisense strand, only the first, second, 9th, 10th and 11th bonds from the 5'-end thereof are phosphorothioate bonds.

**22.** A double-stranded oligonucleotide formed by hybridization of a sense strand oligonucleotide of the following Formula C:

$$5'\text{-N(x)-Consensus Sequence-N(y)-3'} \qquad \text{Formula C}$$

(wherein N(x) is a flanking sequence at the 5'-end, and represents that "N"(s) in the number of x is(are) ligated; N (y) is a flanking sequence at the 3'-end, and represents that "N"(s) in the number of y is(are) ligated; all "N"s each independently represents nucleotide A, G, T, C or U; x and y represent each independently an integer of 1 to 20; and Consensus Sequence represents a consensus sequence to which a transcription factor binds)
and an antisense strand oligonucleotide complementary to said sense strand oligonucleotide, said antisense strand oligonucleotide comprising consecutive 4 bases wherein the bonds therebetween are phosphorothioate bonds, said consecutive 4 bases not including the base at an end of said oligonucleotide.

**23.** The nucleotide according to claim 22, wherein only one set of said consecutive 4 bases is contained in said antisense strand oligonucleotide.

**24.** The nucleotide according to claim 23, wherein only one set of said consecutive 4 bases is contained in said double-stranded nucleotide.

**25.** The nucleotide according to any one of claims 1 to 24, wherein said Consensus Sequence in said Formula C is a binding sequence of NF-κB, E2F, GATA-3, STAT-1, STAT-6, Ets or AP-1.

**26.** The oligonucleotide according to claim 25, wherein said consensus sequence is a binding sequence of NF-κB, said consensus sequence being represented by GGGRHTYYHC (wherein R represents A or G; Y represents C or T; H represents A, C or T) (SEQ ID NO:1).

**27.** The oligonucleotide according to claim 26, wherein said binding sequence of NF-κB is GGGATTTCCC (SEQ ID NO:2) or GGGACTTTCC (SEQ ID NO:3).

**28.** The oligonucleotide according to any one of claims 18 to 27, wherein said Formula C is represented by the following Formula A:

$$5'\text{-N(m)-G-Consensus Sequence -C-N(n)-3'} \qquad \text{(Formula A)}$$

(wherein N(m) is a flanking sequence at the 5'-end, and represents that "N"(s) in the number of m is(are) ligated; N(n) is a flanking sequence at the 3'-end, and represents that "N"(s) in the number of m is(are) ligated; all "N"(s) each independently represents nucleotide A, G, T, C or U; m and n represent each independently an integer of 0 to 20; and the Consensus Sequence represents a consensus sequence to which a transcription factor binds).

**29.** The oligonucleotide according to claim 28, wherein in said Formula A, said m and n are integers of not more than 6.

**30.** The oligonucleotide according to claim 29, wherein in said Formula A, said m is an integer of 2 to 6.

**31.** The oligonucleotide according to any one of claims 22 to 30, wherein in said Formula A, n is an integer of 0, 1, 2 or 3.

**32.** The oligonucleotide according to claim 30 or 31, wherein in said Formula A, N(m) is selected from the group consisting of GA, TGA, TTGA, CTTGA and CCTTGA.

**33.** The oligonucleotide according to claim 32, wherein in said Formula A, N(m) is GA or TTGA, and n is 0.

**34.** The oligonucleotide according to claim 33, wherein said sense strand oligonucleotide is GAGGGGATTTCCCC.

**35.** The oligonucleotide according to claim 34, wherein among bonds between bases in said sense strand GAGGGGATTTCCCC, only the first, second, 10th and 11th bonds from the 5'-end are phosphorothioate bonds, and among bonds between bases in said antisense strand, only the first, 7th, 8th and 9th bonds from the 5'-end are phosphorothioate bonds.

**36.** A transcription factor inhibitor comprising said oligonucleotide according to any one of claims 1 to 35.

**37.** The transcription factor inhibitor according to claim 36, wherein said transcription factor is NF-κB.

**38.** Use of said oligonucleotide according to any one of claims 1 to 35 for the production of a transcription factor inhibitor.

**39.** The use according to claim 38, wherein said transcription factor is NF-κB.

**40.** A method for inhibiting transcription factor in a living body, said method comprising administering to said living body an effective amount of said oligonucleotide according to any one of claims 1 to 35.

**41.** The method according to claim 40, wherein said transcription factor is NF-κB.

**42.** A pharmaceutical composition comprising as an effective ingredient said oligonucleotide according to any one of claims 1 to 35.

**43.** An agent for prophylaxis, amelioration and/or therapy of ischemic diseases, allergic diseases, inflammatory diseases, autoimmune diseases, metastasis/infiltration of cancers, or cachexy, said agent comprising as an effective ingredient said oligonucleotide according to any one of claims 1 to 35.

**44.** An agent for prophylaxis, amelioration and/or therapy of vascular restenosis, acute coronary syndrome, brain ischemia, myocardial infarction, reperfusion hindrance of ischemic diseases, atopic dermatitis, psoriasis vulgaris, contact dermatitis, keloid, decubital ulcer, ulcerative colitis, Crohn's disease, nephropathy, glomerulosclerosis, albuminuria, nephritis, renal failure, rheumatoid arthritis, osteoarthritis, degenerative intervertebral disc, asthma, chronic obstructive pulmonary disease (COPD) or cystic fibrosis (CF), said agent comprising as an effective ingredient said oligonucleotide according to any one of claims 1 to 35.

**45.** An agent for prophylaxis, amelioration and/or therapy of vascular restenosis which occurs after PTCA (percutaneous transluminal coronary angioplasty), PTA (percutaneous transluminal angioplasty), bypass surgery, organ transplantation or surgery of an organ, said agent comprising as an effective ingredient said oligonucleotide according to any one of claims 1 to 35.

**46.** The agent for prophylaxis, amelioration and/or therapy according to claim 45, wherein said vascular restenosis is caused by using an artificial blood vessel, catheter or stent, or by vein grafting.

**47.** The agent for prophylaxis, amelioration and/or therapy according to claim 45, wherein said vascular restenosis is caused by a surgical treatment for arteriosclerosis obliterans, aneurysm, aorta dissection, acute coronary syndrome, brain ischemia, Marfan syndrome or plaque rupture.

**48.** A pharmaceutical composition for prophylaxis, amelioration and/or therapy of a disease(s) caused by NF-κB, said pharmaceutical composition comprising as an effective ingredient said oligonucleotide according to any one of claims 18 to 21, and 25 to 27.

**49.** The pharmaceutical composition according to claim 48, wherein said disease(s) caused by NF-κB is(are) at least one selected from the group consisting of ischemic diseases, allergic diseases, inflammatory diseases, autoimmune diseases, metastasis/infiltration of cancers and cachexy.

**50.** Use of said oligonucleotide according to any one of claims 1 to 35 for the production of a pharmaceutical composition.

**51.** Use of said oligonucleotide according to any one of claims 18 to 21, and 25 to 27 for the production of an agent for therapy or prophylaxis of a disease(s) caused by NF-κB.

**52.** The use according to claim 51, wherein said disease(s) caused by NF-κB is(are) at least one selected from the group consisting of ischemic diseases, allergic diseases, inflammatory diseases, autoimmune diseases, metastasis/infiltration of cancers and cachexy.

**53.** A method for therapy and/or prophylaxis of a disease(s) caused by NF-κB, said method comprising administering to a patient or an animal in need of such therapy and/or prophylaxis an effective amount of said oligonucleotide according to any one of claims 18 to 21, and 25 to 27.

**54.** The method according to claim 53, wherein said disease(s) caused by NF-κB is(are) at least one selected from the group consisting of ischemic diseases, allergic diseases, inflammatory diseases, autoimmune diseases, metastasis/infiltration of cancers and cachexy.

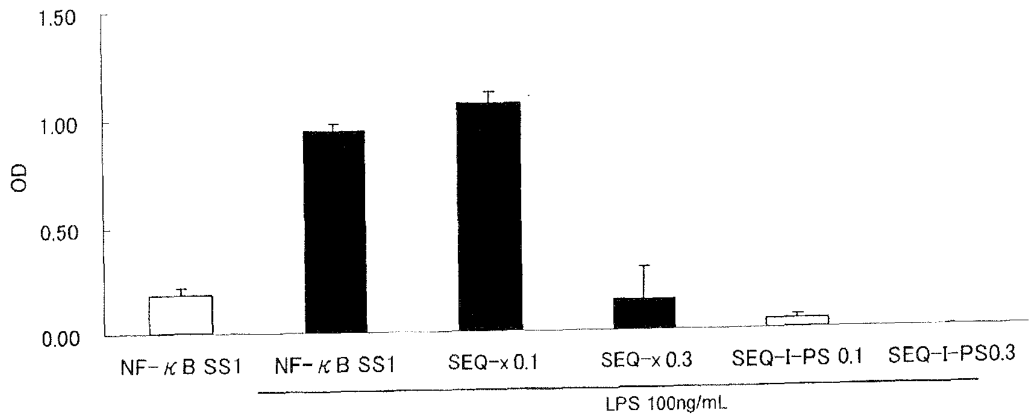


**Fig.1**

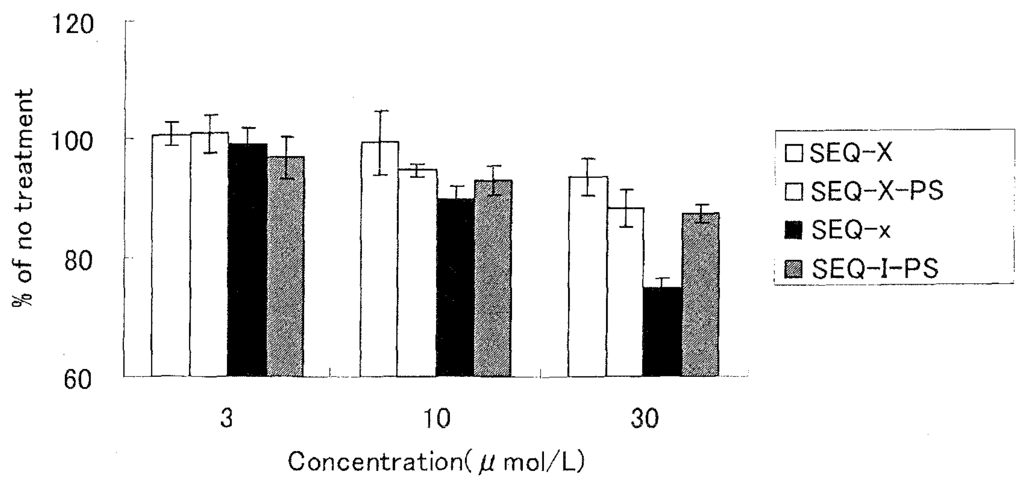


**Fig.2**

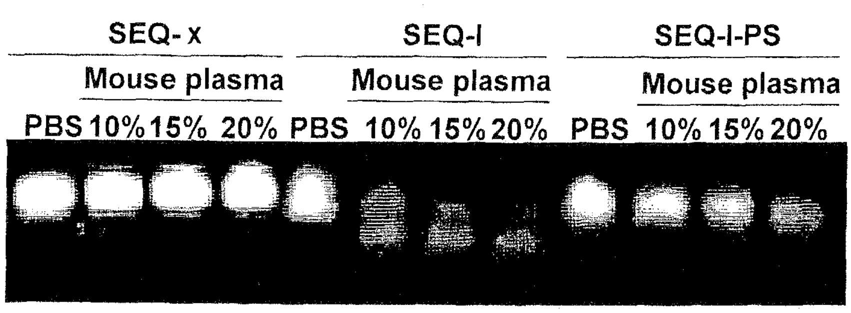


**Fig.3**

**INTERNATIONAL SEARCH REPORT**

International application No. PCT/JP2006/311268

A. CLASSIFICATION OF SUBJECT MATTER
(See extra sheet)

According to International Patent Classification (IPC) or to both national classification and IPC

B. FIELDS SEARCHED

Minimum documentation searched (classification system followed by classification symbols)
C12N15/09, A61K31/713, A61P1/04, A61P9/00, A61P9/10, A61P11/00, A61P11/06, A61P13/02, A61P13/12, A61P17/00, A61P17/02, A61P17/06, A61P19/02, A61P29/00, A61P35/00, A61P35/04, A61P37/00, A61P37/08, A61P41/00, A61P43/00, C07H21/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), BIOSIS/WPI(DIALOG), PubMed, Science Direct, JSTPlus(JDream2), Igaku Yakugaku Yokoshu Zenbun Database, Ichushi WEB

C. DOCUMENTS CONSIDERED TO BE RELEVANT

| Category* | Citation of document, with indication, where appropriate, of the relevant passages | Relevant to claim No. |
|---|---|---|
| P,X | JP 2005-160464 A (Corgentech, Inc.), 23 June, 2005 (23.06.05), Full text & WO 2005/056020 A2 | 1-21,25-52 |
| X<br>Y | QIU J. et al., Effects of NF-kappaB oligonucleotide "decoys" on gene expression in P7 rat hippocampus after hypoxia/ischemia, J.Neurosci.Res., 2004, Vol.77, pages 108 to 118 | 1-5,8-11, 17-19,25-32, 36-52<br>12-16 |
| X<br>Y | JP 2004-298178 A (Corgentech, Inc.), 28 October, 2004 (28.10.04), Full text & WO 2004/087965 A2 & US 2004/191779 A1 108-118 | 1,2,4,17-19, 25-29,31, 36-52<br>8-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 September, 2006 (06.09.06) | 12 September, 2006 (12.09.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/311268

C (Continuation). DOCUMENTS CONSIDERED TO BE RELEVANT

| Category* | Citation of document, with indication, where appropriate, of the relevant passages | Relevant to claim No. |
|---|---|---|
| Y | LEVIN A.A. et al., A review of the issues in the pharmacokinetics and toxicology of phosphorothioate antisense oligonucleotides, Biochim. Biophys. Acta., 1999, vol.1489, p.69-84 | 8-16 |
| A | LA FERLA K. et al., Inhibition of erythropoietin gene expression signaling involves the transcription factors GATA-2 and NF-kappaB, FASEB J., 2002, vol.16, p.1811-1813, Epub 2002 Sep. 5 | 1-21,25-52 |
| A | WO 2004/026342 A1 (Anges MG, Inc.), 01 April, 2004 (01.04.04), Full text (Family: none) | 1-21,25-52 |

Form PCT/ISA/210 (continuation of second sheet) (April 2005)

**INTERNATIONAL SEARCH REPORT**

International application No.
PCT/JP2006/311268

Continuation of A. CLASSIFICATION OF SUBJECT MATTER (International Patent Classification (IPC))

*C12N15/09*(2006.01)i, *A61K31/713*(2006.01)i, *A61P1/04*(2006.01)i, *A61P9/00*(2006.01)i, *A61P9/10*(2006.01)i, *A61P11/00*(2006.01)i, *A61P11/06*(2006.01)i, *A61P13/02*(2006.01)i, *A61P13/12*(2006.01)i, *A61P17/00*(2006.01)i, *A61P17/02*(2006.01)i, *A61P17/06*(2006.01)i, *A61P19/02*(2006.01)i, *A61P29/00*(2006.01)i, *A61P35/00*(2006.01)i, *A61P35/04*(2006.01)i, *A61P37/00*(2006.01)i, *A61P37/08*(2006.01)i, *A61P41/00*(2006.01)i, *A61P43/00*(2006.01)i, *C07H21/00*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2006/311268

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 53, 54
because they relate to subject matter not required to be searched by this Authority, namely:
The inventions as set forth in claims 53 and 54 are relevant to "methods for treatment of the human body by surgery or therapy and diagnostic methods".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
The inventions of claims 1-21 and parts of the inventions of claims 25-54 (invention group A) relate to a double-stranded oligonucleotide composed of a sense oligonucleotide having a nucleotide sequence of the formula A and a complementary sequence thereof. The inventions of claims 22-24 and parts of the inventions of claims 25-54 (invention group B) relate to a double-stranded oligonucleotide composed of a sense oligonucleotide having a nucleotide sequence of the formula C and a complementary sequence thereof.
The technical matter common to the invention groups A and B obviously resides in a double-stranded oligonucleotide composed of a sense oligonucleotide having

(continued to extra sheet)

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-21 and parts of 25-52

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

Form PCT/ISA/210 (continuation of first sheet (2)) (April 2005)

**INTERNATIONAL SEARCH REPORT**

International application No.
PCT/JP2006/311268

Continuation of Box No.III of continuation of first sheet(2)

a nucleotide sequence which has 1 to 20 nucleotides added to both termini of a consensus sequence and a complementary sequence thereof. However, this technical matter is known at the time of the priority date of the present application, as disclosed in JP 2004-298178 A, WO 2004/026342 A and the like. Thus, this common matter cannot be regarded as a special technical feature in the meaning within PCT Rule 13.2, second sentence.

Thus, the present international application does not comply with the requirement of unity of inventions.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 8035430 A
- JP 3392143 B
- WO 9511687 A
- JP 8501928 A
- WO 9635430 A
- WO 02066070 A
- WO 03043663 A
- WO 03082331 A
- WO 03099339 A
- WO 04026342 A
- WO 05004913 A
- WO 05004914 A
- JP 2001286282 A


### Non-patent literature cited in the description


- **MILLIGAN et al.** *J. Med. Chem.,* 1993, vol. 36, 1923
- **STEIN ; CHENG.** *Science,* 1993, vol. 261, 1004
- **LEVIN et al.** *Biochem. Biophys. Acta,* 1999, vol. 1489, 69
- *FASEB J,* 16 November 2002, (13), 1811-3
- *Naunyu-Schmiedeberg's Arch Pharmacol,* 2001, vol. 364, 422-429
- **MARINA A. et al.** *Journal of Biological Chemistry,* 1995, vol. 270 (6), 2620-2627